# EUROPEAN PATENT APPLICATION

(11) **EP 4 613 738 A1**
(43) Date of publication of application: **10.09.2025**
(21) Application number: 23886104.1
(22) Date of filing: 20.10.2023
(51) Int. Cl.: C07C 237/06, C07C 231/14

(54) **LIPID HAVING AMIDE AND ESTER FUNCTIONAL GROUPS, AND METHOD FOR PRODUCING SAME**

(30) Priority: 01.11.2022 KR 20220143883
(71) Applicant: Samyang Holdings Corporation, Seoul 03129 (KR)
(72) Inventor: PARK, Jong Min, Seongnam-si Gyeonggi-do 13590 (KR); SEONG, Shi Hwa, Seoul 06008 (KR); KYUNG, Kyu Jin, Yongin-si Gyeonggi-do 16827 (KR); YOO, Seung Ju, Seoul 07943 (KR); YOON, Yoo Jeong, Seongnam-si Gyeonggi-do 13544 (KR); LEE, So Jin, Seoul 02468 (KR); HAN, Ji Hye, Seoul 03733 (KR); KIM, Bong Oh, Daejeon 35212 (KR); NAM, Joung Pyo, Seoul 02471 (KR)
(74) Representative: Gleiss Große Schrell und Partner mbB
(86) International application number: PCT/KR2023/016408
(87) International publication number: WO 2024/096408

(57) **Abstract**

The present invention pertains to a lipid having amide and ester functional groups, and a method for producing same. More specifically, the present invention pertains to: an ionized lipid that forms a polyplex with an anionic drug and is useful for drug delivery due to a specific structure having amide and ester functional groups; and a method for producing same.

## Description

### TECHNICAL FIELD

The present invention relates to a lipid having amide and ester functional groups and a method for preparing the same, and more specifically, the present invention relates to an ionizable lipid which forms a complex with anionic drug and thus is useful for drug delivery due to its specific structure having amide and ester functional groups, and a method for preparing the same.

### BACKGROUND ART

In therapies using anionic drugs including nucleic acid, technologies for safe and efficient drug delivery have been researched for a long time, and various carriers and techniques for delivery have been developed. Carriers are mainly divided into viral carriers utilizing adenovirus, retrovirus or the like, and non-viral carriers utilizing cationic lipid, cationic polymer or the like. Viral carriers are known as being exposed to risks such as non-specific immune response, etc. and having many problems in commercialization due to the complexity of the production process. Thus, recent researches proceed in the direction to improve such disadvantages by using non-viral carriers. In comparison with viral carriers, non-viral carriers have advantages of fewer side effects in terms of *in vivo* safety, and lower production cost in terms of economy.

The representative non-viral carriers for delivering nucleic acid material are a complex of cationic lipid and nucleic acid (lipoplex) and a complex of polycationic polymer and nucleic acid (polyplex). Such a cationic lipid or polycationic polymer stabilizes anionic drug by forming a complex through electrostatic interaction with the anionic drug and increases intracellular delivery, and for these reasons, various researches thereof have been conducted (De Paula D, Bentley MV, Mahato RI, Hydrophobization and bioconjugation for enhanced siRNA delivery and targeting, RNA 13 (2007) 431-56; Gary DJ, Puri N, Won YY, Polymer-based siRNA delivery: Perspectives on the fundamental and phenomenological distinctions from polymer-based DNA delivery, J Control release 121 (2007) 64-73).

However, polycation polymers have cytotoxicity due to their multivalent cationic charges, making them problematic for practical use, and nucleic acid-cationic lipid complexes have low stability in the blood, making them difficult to use *in vivo* actually. In addition, ionic liposomes including cationic lipid, neutral lipid, and fusogenic lipid have the disadvantages of complication in method of synthesizing the cationic lipid used, cytotoxicity, and low efficiency of intracellular nucleic acid delivery.

### CONTENTS OF THE INVENTION

### PROBLEMS TO BE SOLVED

The purpose of the present invention is to provide a lipid having a specific structure which can easily form a complex with anionic drug and thus is useful for drug delivery, and a method for preparing the same.

### TECHNICAL MEANS

The first aspect of the present invention provides a lipid having a structure selected from the following, or an ionized form thereof: 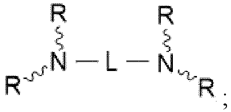 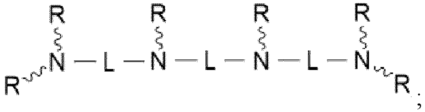 and 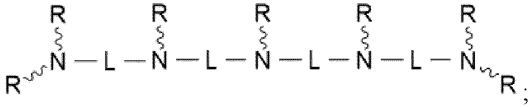 wherein, in each of the above structures,
at least two of R groups are Rx, and other R groups are Ry, wherein
each Rx is independently selected from 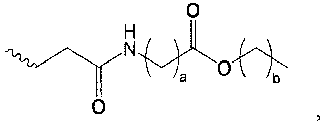 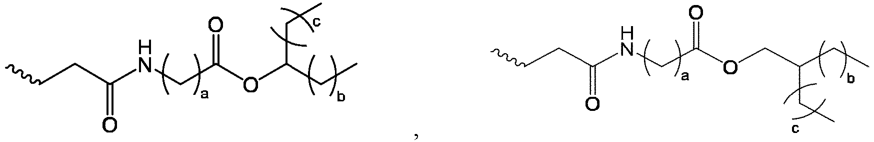 and 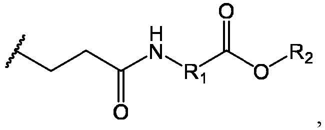 where each of a, b and c is independently an integer of from 2 to 20, R₁ is substituted or unsubstituted, saturated or unsaturated divalent hydrocarbon group, R₂ is substituted or unsubstituted, unsaturated monovalent hydrocarbon group, and 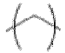 represents substituted or unsubstituted methylene group, and
each Ry is independently H, or substituted or unsubstituted alkyl group, where two Ry groups that are not H may be connected together with nitrogen atom to which they are attached, to form a ring structure; and
each L is independently substituted or unsubstituted alkylene group, and may have in its structure optionally ether bond (-O-), thioether bond (-S-) or disulfide bond (-S-S-).

According to an embodiment of the present invention, each Ry may be independently H or C₁₋₂₀ alkyl group, where the alkyl group may be independently unsubstituted, or may be substituted with one or more selected from -OH, C₁₋₂₀ alkyl, C₁₋₂₀ alkoxy, -NH₂, -NH(C₁₋₂₀ alkyl), -N(C₁₋₂₀ alkyl)₂, optionally substituted C₃₋₂₀ carbocyclic group and optionally substituted C₃₋₂₀ heterocyclic group, where the heterocyclic group may have one or more (e.g., 1 to 3) heteroatoms selected from N, O and S; and two Ry groups that are not H may be connected together with nitrogen atom to which they are attached, to form a ring structure optionally having one or more heteroatoms selected from N and O.

According to an embodiment of the present invention, each L is independently C₁₋₂₀ alkylene group, each of which may be independently unsubstituted, or may be substituted with one or more selected from -OH, C₁₋₂₀ alkyl, C₁₋₂₀ alkoxy, -NH₂, -NH(C₁₋₂₀ alkyl), -N(C₁₋₂₀ alkyl)₂, optionally substituted C₃₋₂₀ carbocyclic group and optionally substituted C₃₋₂₀ heterocyclic group, where the heterocyclic group may have one or more (e.g., 1 to 3) heteroatoms selected from N, O and S.

More concretely, each Rx is independently selected from 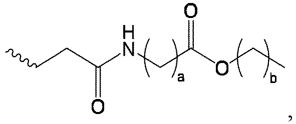 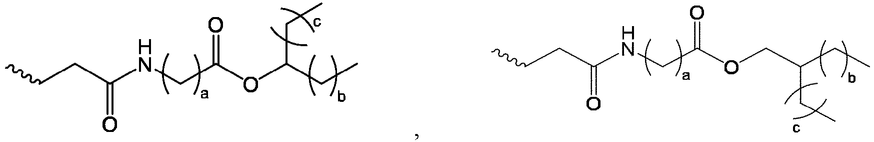 and 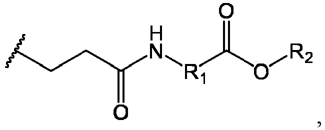 where each of a, b and c may be independently an integer of from 2 to 20 or from 2 to 15, R₁ may be substituted or unsubstituted, saturated or unsaturated divalent C₁₋₁₂ hydrocarbon group, R₂ may be substituted or unsubstituted, unsaturated monovalent C₂₋₂₄ hydrocarbon group, and 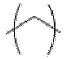 represents substituted or unsubstituted methylene group.

More concretely, each Ry may be independently H or C₁₋₁₀ alkyl group, where the alkyl group may be independently unsubstituted, or may be substituted with one or more selected from -OH, C₁₋₁₀ alkyl, C₁₋₁₀ alkoxy, -NH₂, -NH(C₁₋₁₀ alkyl), -N(C₁₋₁₀ alkyl)₂, optionally substituted C₃₋₁₀ carbocyclic group and optionally substituted C₃₋₁₀ heterocyclic group, where the heterocyclic group may have one or more (e.g., 1 to 3) heteroatoms selected from N, O and S; and two Ry groups that are not H may be connected together with nitrogen atom to which they are attached, to form a ring structure optionally having one or more heteroatoms selected from N and O.

More concretely, each L may be independently C₁₋₁₀ alkylene group, each of which may be independently unsubstituted, or may be substituted with one or more selected from -OH, C₁₋₁₀ alkyl, C₁₋₁₀ alkoxy, -NH₂, -NH(C₁₋₁₀ alkyl), -N(C₁₋₁₀ alkyl)₂, optionally substituted C₃₋₁₀ carbocyclic group and optionally substituted C₃₋₁₀ heterocyclic group, where the heterocyclic group may have one or more (e.g., 1 to 3) heteroatoms selected from N, O and S.

Even more concretely, each Rx is independently selected from 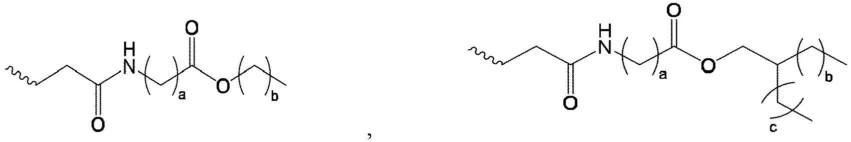 and 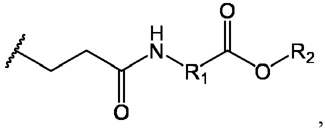 where each of a, b and c may be independently an integer of from 3 to 12, R₁ may be substituted or unsubstituted C₁₋₁₂ alkylene group, substituted or unsubstituted C₂₋₁₂ alkenylene group or substituted or unsubstituted C₂₋₁₂ alkynylene group, R₂ may be substituted or unsubstituted C₂₋₂₄ alkenyl group or substituted or unsubstituted C₂₋₂₄ alkynyl group, and 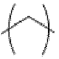 represents substituted or unsubstituted methylene group.

Even more concretely, each Ry may be independently H or C₁₋₆ alkyl group, where the alkyl group may be independently unsubstituted, or may be substituted with one or more selected from -OH and -NH₂; and two Ry groups that are not H may be connected together with nitrogen atom to which they are attached, to form a ring structure optionally having one or more heteroatoms selected from N and O.

Even more concretely, each L may be independently unsubstituted C₁₋₆ alkylene group.

Still more concretely, the lipid may be one having a structure selected from the following formulas A to V:

| Formula | Structure |
|---|---|
| A | 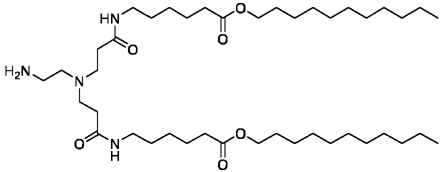 |
| B | 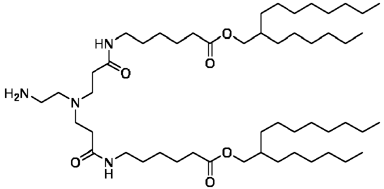 |
| C | 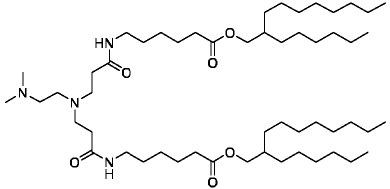 |
| D | 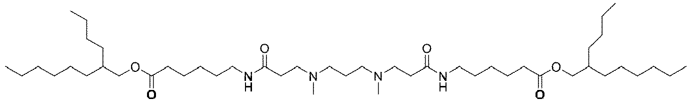 |
| E | 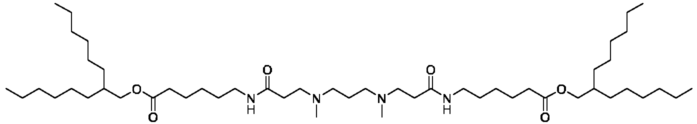 |
| F | 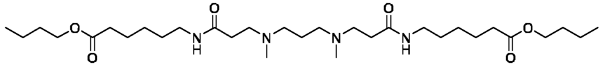 |
| G | 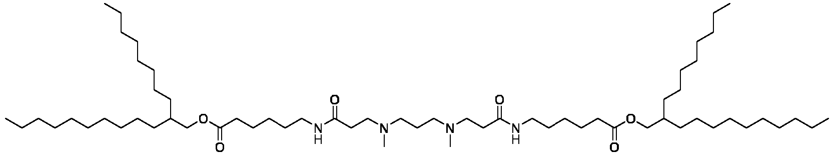 |
| H | 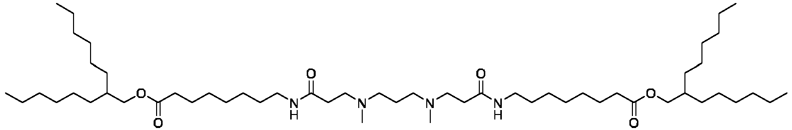 |
| I | 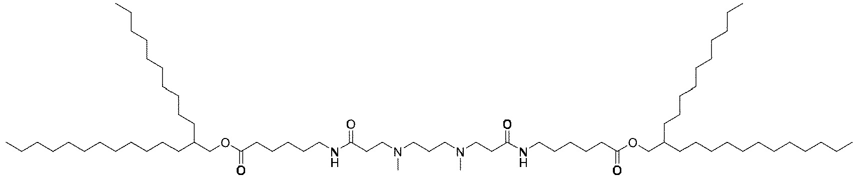 |
| J | 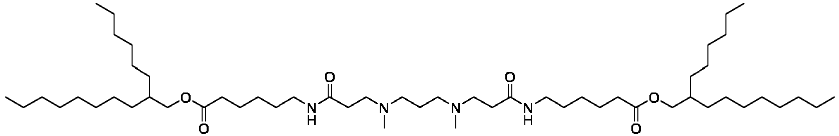 |
| K | 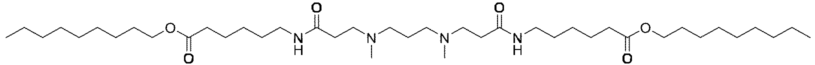 |
| L | 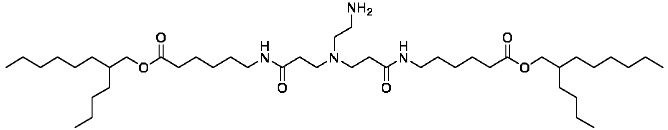 |
| M | 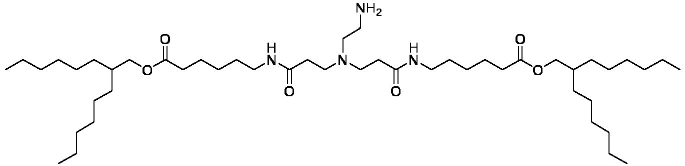 |
| N | 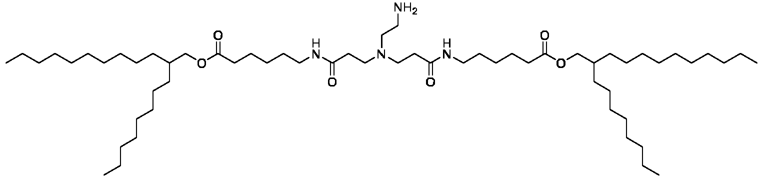 |
| O | 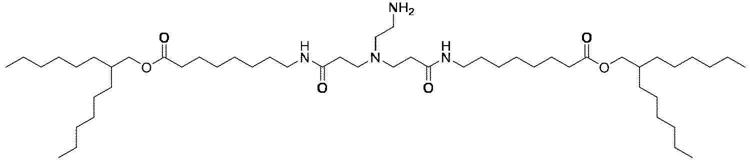 |
| P | 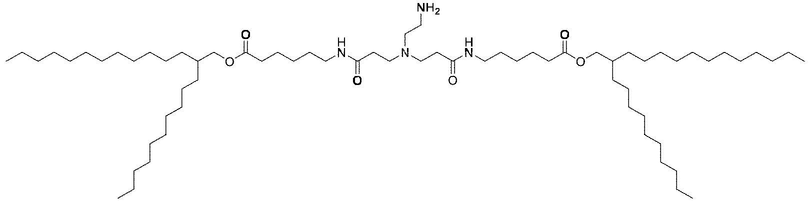 |
| Q | 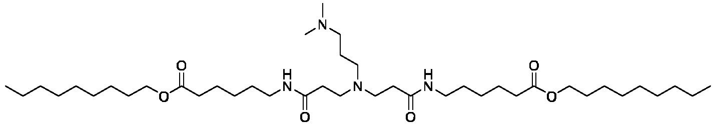 |
| R | 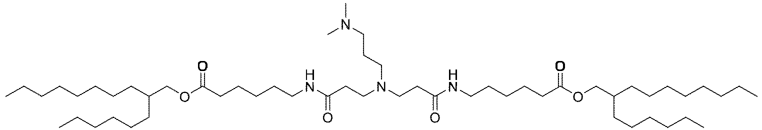 |
| S | 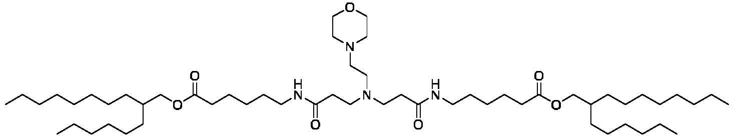 |
| T | 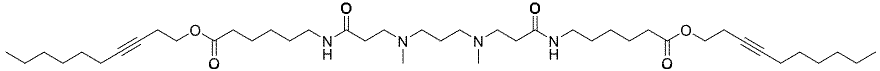 |
| U | 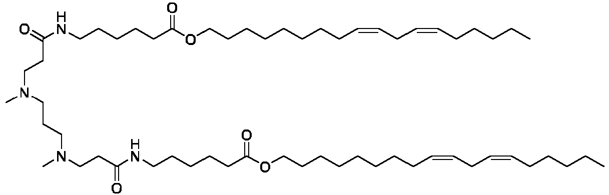 |
| V | 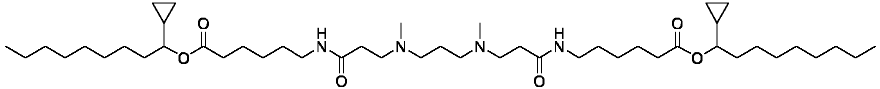 |

The second aspect of the present invention provides a method for preparing a lipid having a structure represented by formula (1-1), comprising steps of: (1) reacting a compound of formula (a) with a compound of formula (b) to obtain a compound of formula (c); (2) reacting a compound of formula (c) with a compound of formula (d) to obtain a compound of formula (e); and (3) reacting a compound of formula (e) with a compound of formula (f) and deprotecting the reaction product:

[Formula a] H₂N-(CH₂)ₐ-C(=O)OH

[Formula b] OH-R'

[Formula c] H₂N-(CH₂)ₐ-C(=O)O-R'

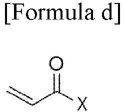

[Formula e] H₂C=CH-C(=O)-HN-(CH₂)ₐ-C(=O)O-R'

[Formula f] H₂N-(CH₂)₁₋₂₀-NH-C(=O)O-C(CH₃)₃

[Formula 1-1] H₂N-(CH₂)₁₋₂₀-N[-CH₂-CH₂-C(=O)-HN-(CH₂)ₐ-C(=O)O-R']₂

wherein,
each R' is independently 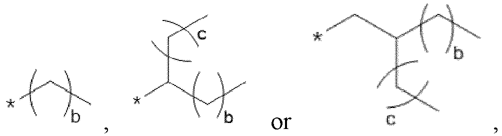 where * indicates the point of attachment to the adjacent oxygen atom, and 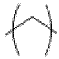 represents substituted or unsubstituted methylene group,
each of a, b and c is independently an integer of from 2 to 20, and
X is selected from the group consisting of F, CI, Br and I.

The third aspect of the present invention provides a method for preparing a lipid having a structure represented by formula (1-2), comprising a step of reacting a compound of formula (e) obtained from the second aspect of the present invention with a compound of formula (g):

[Formula e] H₂C=CH-C(=O)-HN-(CH₂)ₐ-C(=O)O-R'

[Formula g] H₂N-(CH₂)₁₋₂₀-N(C₁₋₂₀ alkyl)₂

[Formula 1-2] (C₁₋₂₀ alkyl)₂N-(CH₂)₁₋₂₀-N[-CH₂-CH₂-C(=O)-HN-(CH₂)ₐ-C(=O)O-R']₂

wherein,
each R' is independently 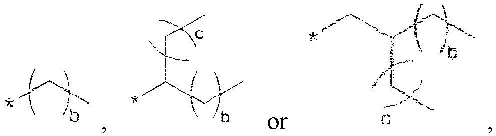 where * indicates the point of attachment to the adjacent oxygen atom, and 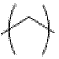 represents substituted or unsubstituted methylene group, and
each of a, b and c is independently an integer of from 2 to 20.

The fourth aspect of the present invention provides a method for preparing a lipid having a structure represented by formula (1-3), comprising a step of reacting a compound of formula (e) obtained from the second aspect of the present invention with a compound of formula (h):

[Formula e] H₂C=CH-C(=O)-HN-(CH₂)ₐ-C(=O)O-R'

[Formula h] (C₁₋₁₀ alkyl)-NH-(CH₂)₁₋₂₀-NH-(C₁₋₁₀ alkyl)

[Formula 1-3] A-N(C₁₋₁₀ alkyl)-(CH₂)₁₋₂₀-N(C₁₋₁₀ alkyl)-A

wherein,
each R' is independently 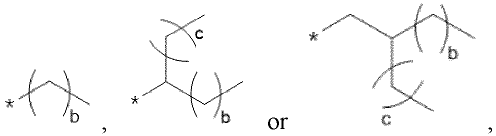 where * indicates the point of attachment to the adjacent oxygen atom, and 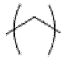 represents substituted or unsubstituted methylene group,
A is -CH₂-CH₂-C(=O)-HN-(CH₂)ₐ-C(=O)O-R',
each of a, b and c is independently an integer of from 2 to 20, and
X is selected from the group consisting of F, CI, Br and I.

The fifth aspect of the present invention provides a method for preparing a lipid having a structure represented by formula (1-4), comprising steps of: (1) reacting a compound of formula (a) with a compound of formula (b') to obtain a compound of formula (c'); (2) reacting a compound of formula (c') with a compound of formula (d) to obtain a compound of formula (e'); and (3) reacting a compound of formula (e') with a compound of formula (h):

[Formula a] H₂N-(CH₂)ₐ-C(=O)OH

[Formula b'] OH-R₂

[Formula c'] H₂N-R₁-C(=O)O-R₂

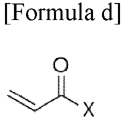

[Formula e'] H₂C=CH-C(=O)-HN-R₁-C(=O)O-R₂

[Formula h] (C₁₋₁₀ alkyl)-NH-(CH₂)₁₋₂₀-NH-(C₁₋₁₀ alkyl)

[Formula 1-4] A'-N(C₁₋₁₀ alkyl)-(CH₂)₁₋₂₀-N(C₁₋₁₀ alkyl)-A'

wherein,
A' is -CH₂-CH₂-C(=O)-HN-R₁-C(=O)O-R₂,
R₁ is independently substituted or unsubstituted, saturated or unsaturated divalent hydrocarbon group,
R₂ is independently substituted or unsubstituted, unsaturated monovalent hydrocarbon group,
a is an integer of from 2 to 20, and
X is selected from the group consisting of F, CI, Br and I.

The sixth aspect of the present invention provides a composition for drug delivery comprising the lipid according to the present invention.

### EFFECT OF THE INVENTION

The lipid having a specific structure according to the present invention can easily form a complex with anionic drug, and by utilizing the complex, the drug can be efficiently delivered into the targeted living tissue.

### BRIEF EXPLANATION OF THE DRAWINGS

Figure 1 is a reaction scheme for the lipid synthesis procedure conducted in Example 1.
Figure 2 is a reaction scheme for the lipid synthesis procedure conducted in Example 2.
Figure 3 is a reaction scheme for the lipid synthesis procedure conducted in Example 3.
Figure 4 is a reaction scheme for the lipid synthesis procedure conducted in Example 4.
Figure 5 is a reaction scheme for the lipid synthesis procedure conducted in Example 5.
Figure 6 is a reaction scheme for the lipid synthesis procedure conducted in Example 6.
Figure 7 is a reaction scheme for the lipid synthesis procedure conducted in Example 7.
Figure 8 is a reaction scheme for the lipid synthesis procedure conducted in Example 8.
Figure 9 is a reaction scheme for the lipid synthesis procedure conducted in Example 9.
Figure 10 is a reaction scheme for the lipid synthesis procedure conducted in Example 10.
Figure 11 is a reaction scheme for the lipid synthesis procedure conducted in Example 11.
Figure 12 is a reaction scheme for the lipid synthesis procedure conducted in Example 12.
Figure 13 is a reaction scheme for the lipid synthesis procedure conducted in Example 13.
Figure 14 is a reaction scheme for the lipid synthesis procedure conducted in Example 14.
Figure 15 is a reaction scheme for the lipid synthesis procedure conducted in Example 15.
Figure 16 is a reaction scheme for the lipid synthesis procedure conducted in Example 16.
Figure 17 is a reaction scheme for the lipid synthesis procedure conducted in Example 17.
Figure 18 is a reaction scheme for the lipid synthesis procedure conducted in Example 18.
Figure 19 is a reaction scheme for the lipid synthesis procedure conducted in Example 19.
Figure 20 is a reaction scheme for the lipid synthesis procedure conducted in Example 20.
Figure 21 is a reaction scheme for the lipid synthesis procedure conducted in Example 21.
Figure 22 is a reaction scheme for the lipid synthesis procedure conducted in Example 22.

### CONCRETE MODE FOR CARRYING OUT THE INVENTION

The present invention will be explained in detail below.

A lipid provided by the first aspect of the present invention has a structure selected from the following, or is an ionized form thereof: 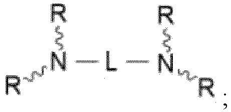 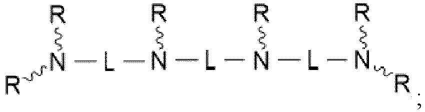 and 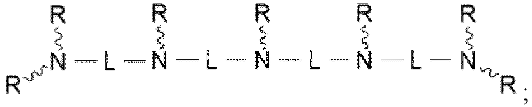 wherein, in each of the above structures,
at least two (more concretely, 2 to 7) of R groups are Rx, and other R groups are Ry, wherein
each Rx is independently selected from 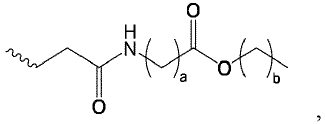 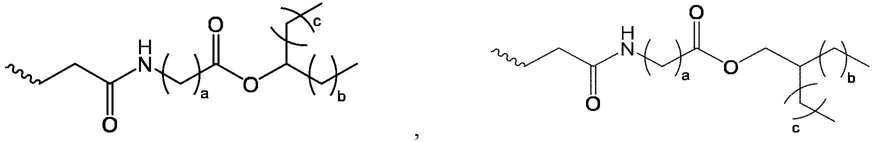 and 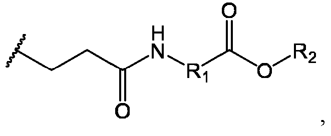 where each of a, b and c is independently an integer of from 2 to 20, R₁ is substituted or unsubstituted, saturated or unsaturated divalent hydrocarbon group, R₂ is substituted or unsubstituted, unsaturated monovalent hydrocarbon group, and 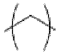 represents substituted or unsubstituted methylene group, and
each Ry is independently H, or substituted or unsubstituted alkyl group, where two Ry groups that are not H may be connected together with nitrogen atom to which they are attached, to form a ring structure; and
each L is independently substituted or unsubstituted alkylene group, and may have in its structure optionally ether bond (-O-), thioether bond (-S-) or disulfide bond (-S-S-).

As used herein, the expression "substituted or unsubstituted" for any group means that, unless specified otherwise, the group is not substituted, or is substituted with one or mor substituents selected from -OH, halogen atom, C₁₋₆ alkyl group, C₁₋₆ alkoxy group, C₁₋₆ halogenated alkyl group, C₁₋₆ halogenated alkoxy group, C₃₋₂₀ cycloalkyl group, C₃₋₂₀ heterocycloalkyl group, C₆₋₂₀ aryl group or C₃₋₂₀ heteroaryl group.

According to an embodiment of the present invention, each Rx is independently selected from 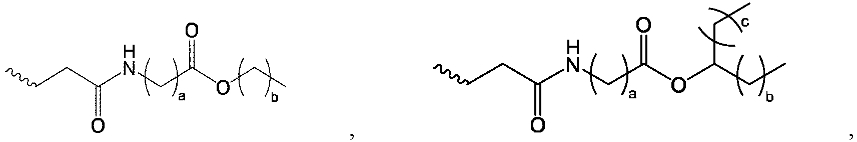 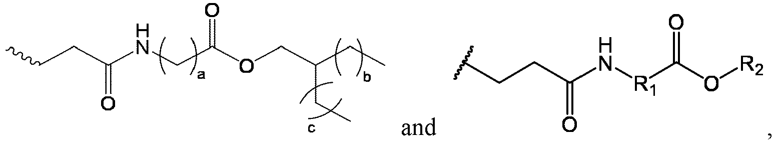 where each of a, b and c may be independently an integer of from 2 to 20 or from 2 to 15, R₁ may be substituted or unsubstituted, saturated or unsaturated divalent C₁₋₁₂ hydrocarbon group, R₂ may be substituted or unsubstituted, unsaturated monovalent C₂₋₂₄ hydrocarbon group, and 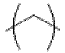 represents substituted or unsubstituted methylene group.

According to an embodiment of the present invention, each of a, b and c may be independently an integer of from 2 to 15, and more concretely, may be independently an integer of from 3 to 12. Even more concretely, a may be independently an integer of from 5 to 7, and each of b and c may be independently an integer of from 3 to 11, but it is not limited thereto.

According to an embodiment of the present invention, each Ry may be independently H or C₁₋₂₀ alkyl group, where the alkyl group may be independently unsubstituted, or may be substituted with one or more selected from -OH, C₁₋₂₀ alkyl, C₁₋₂₀ alkoxy, -NH₂, -NH(C₁₋₂₀ alkyl), -N(C₁₋₂₀ alkyl)₂, optionally substituted C₃₋₂₀ carbocyclic group (e.g., C₃₋₂₀ cycloalkyl group or C₆₋₂₀ aryl group) and optionally substituted C₃₋₂₀ heterocyclic group (e.g., C₃₋₂₀ heterocycloalkyl group or C₃₋₂₀ heteroaryl group), where the heterocyclic group may have one or more (e.g., 1 to 3) heteroatoms selected from N, O and S; and two Ry groups that are not H may be connected together with nitrogen atom to which they are attached, to form a ring structure optionally having one or more heteroatoms selected from N and O. Also, in the above, the alkyl or alkoxy group may be more concretely C₁₋₁₀ alkyl or alkoxy group, and still more concretely C₁₋₆ alkyl or alkoxy group, but it is not limited thereto.

According to an embodiment of the present invention, each L is independently C₁₋₂₀ alkylene group (more concretely C₁₋₁₀ alkylene group, and still more concretely C₁₋₆ alkylene group), each of which may be independently unsubstituted, or may be substituted with one or more selected from -OH, C₁₋₂₀ alkyl, C₁₋₂₀ alkoxy, -NH₂, -NH(C₁₋₂₀ alkyl), -N(C₁₋₂₀ alkyl)₂, optionally substituted C₃₋₂₀ carbocyclic group (e.g., C₃₋₂₀ cycloalkyl group or C₆₋₂₀ aryl group) and optionally substituted C₃₋₂₀ heterocyclic group (e.g., C₃₋₂₀ heterocycloalkyl group or C₃₋₂₀ heteroaryl group), where the heterocyclic group may have one or more (e.g., 1 to 3) heteroatoms selected from N, O and S. Also, in the above, the alkyl or alkoxy group may be more concretely C₁₋₁₀ alkyl or alkoxy group, and still more concretely C₁₋₆ alkyl or alkoxy group, but it is not limited thereto.

More concretely, each Ry may be independently H or C₁₋₁₀ alkyl group, where the alkyl group may be independently unsubstituted, or may be substituted with one or more selected from -OH, C₁₋₁₀ alkyl, C₁₋₁₀ alkoxy, -NH₂, -NH(C₁₋₁₀ alkyl), -N(C₁₋₁₀ alkyl)₂, optionally substituted C₃₋₁₀ carbocyclic group and optionally substituted C₃₋₁₀ heterocyclic group, where the heterocyclic group may have one or more (e.g., 1 to 3) heteroatoms selected from N, O and S; and two Ry groups that are not H may be connected together with nitrogen atom to which they are attached, to form a ring structure optionally having one or more heteroatoms selected from N and O.

More concretely, each L may be independently C₁₋₁₀ alkylene group, each of which may be independently unsubstituted, or may be substituted with one or more selected from -OH, C₁₋₁₀ alkyl, C₁₋₁₀ alkoxy, -NH₂, -NH(C₁₋₁₀ alkyl), -N(C₁₋₁₀ alkyl)₂, optionally substituted C₃₋₁₀ carbocyclic group and optionally substituted C₃₋₁₀ heterocyclic group, where the heterocyclic group may have one or more (e.g., 1 to 3) heteroatoms selected from N, O and S.

Even more concretely, each Rx is independently selected from 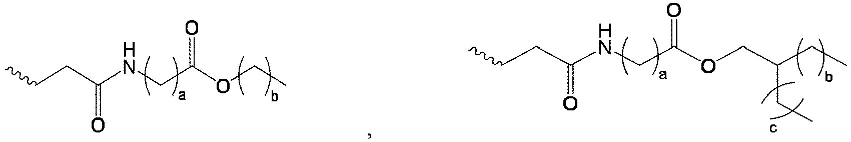 and 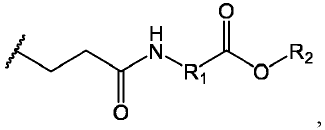 where each of a, b and c may be independently an integer of from 3 to 12, R₁ may be substituted or unsubstituted C₁₋₁₂ alkylene group, substituted or unsubstituted C₂₋₁₂ alkenylene group or substituted or unsubstituted C₂₋₁₂ alkynylene group, R₂ may be substituted or unsubstituted C₂₋₂₄ alkenyl group or substituted or unsubstituted C₂₋₂₄ alkynyl group, and 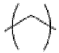 represents substituted or unsubstituted methylene group.

Even more concretely, each Ry may be independently H or C₁₋₆ alkyl group, where the alkyl group may be independently unsubstituted, or may be substituted with one or more selected from -OH and -NH₂; and two Ry groups that are not H may be connected together with nitrogen atom to which they are attached, to form a ring structure optionally having one or more heteroatoms selected from N and O.

Even more concretely, each L may be independently unsubstituted C₁₋₆ alkylene group.

Concretely, the lipid may have a structure selected from the following:

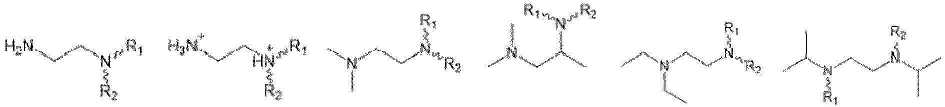

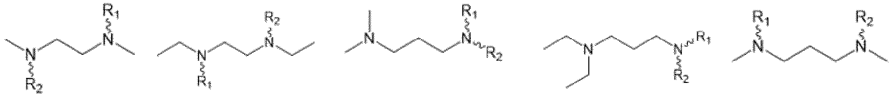

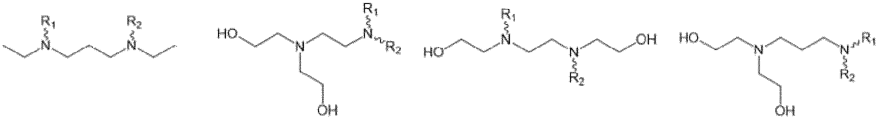

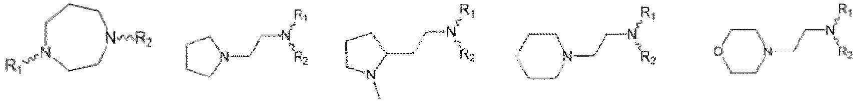

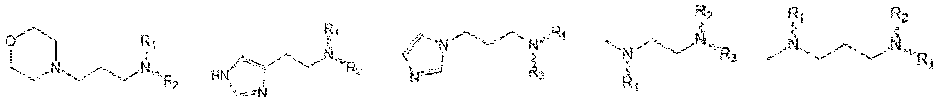

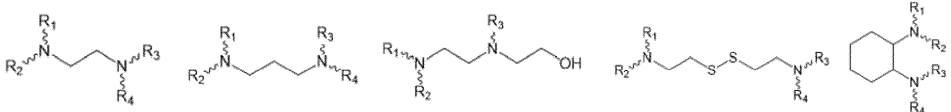

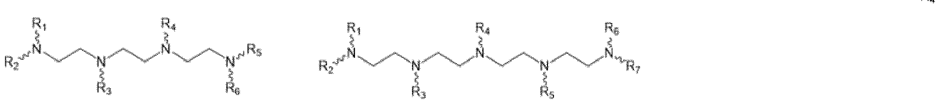

In each of the above structures, each of R₁ to R₇ is independently selected from 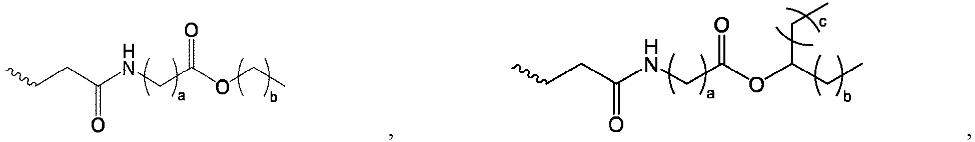 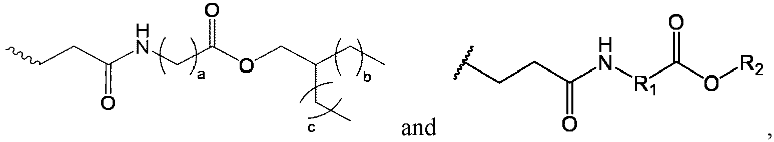 where each of a, b and c is independently an integer of from 2 to 20, R₁ is substituted or unsubstituted, saturated or unsaturated divalent hydrocarbon group, R₂ is substituted or unsubstituted, unsaturated monovalent hydrocarbon group, and 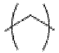 represents substituted or unsubstituted methylene group

Still more concretely, the lipid may be one having a structure selected from the following formulas A to V:

| Formula | Structure |
|---|---|
| A | 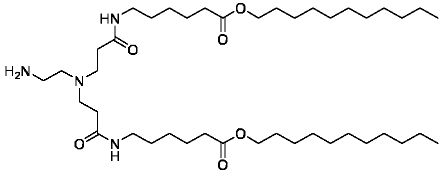 |
| B | 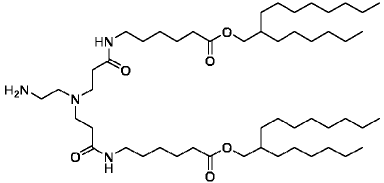 |
| C | 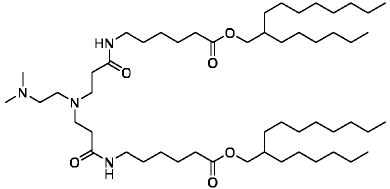 |
| D | 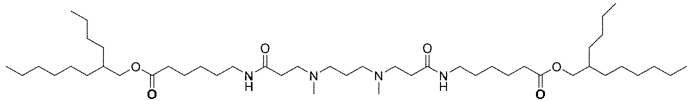 |
| E | 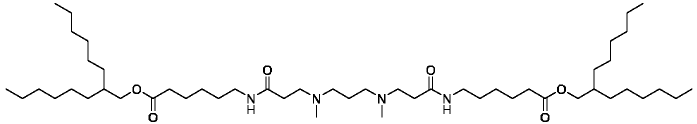 |
| F | 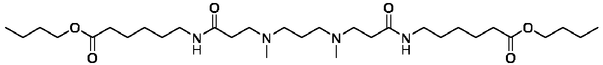 |
| G | 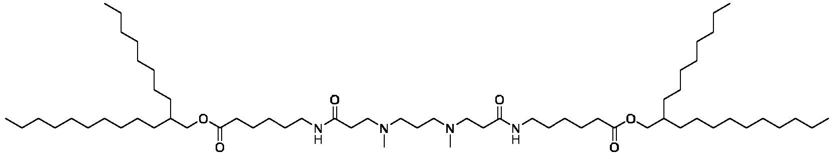 |
| H | 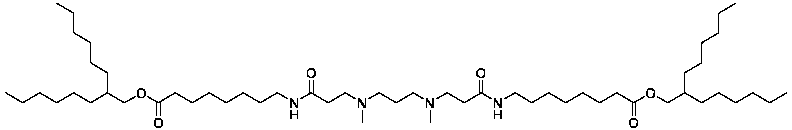 |
| I | 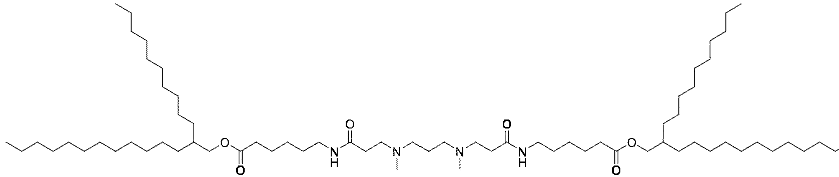 |
| J | 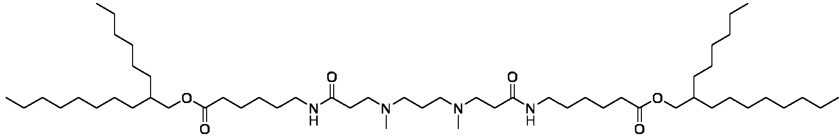 |
| K | 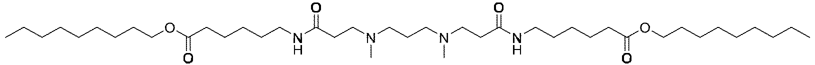 |
| L | 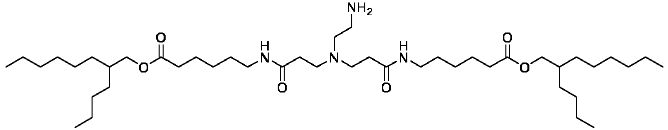 |
| M | 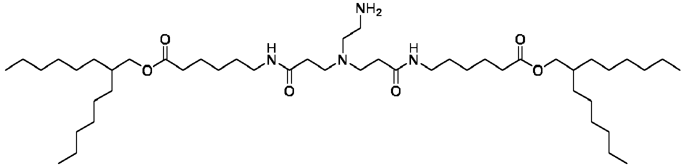 |
| N | 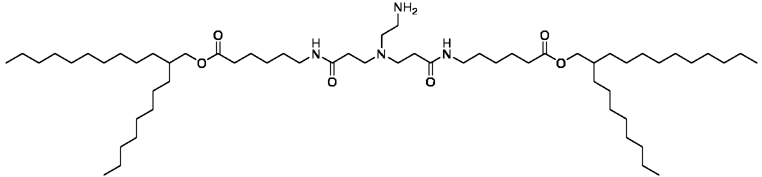 |
| O | 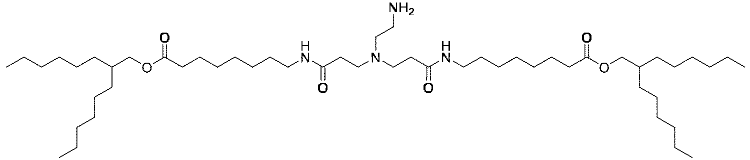 |
| P | 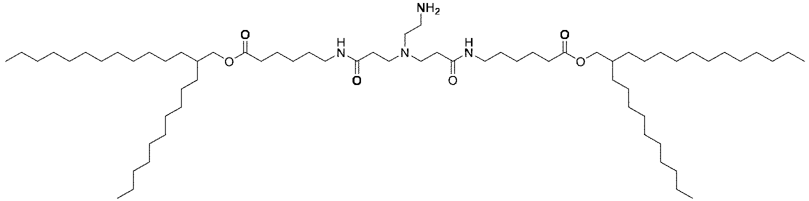 |
| Q | 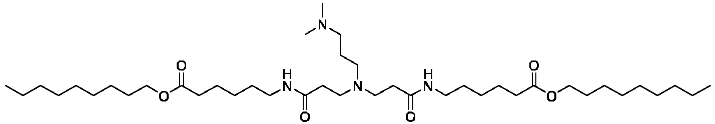 |
| R | 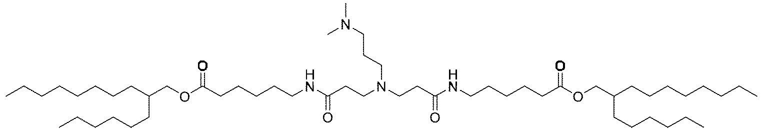 |
| S | 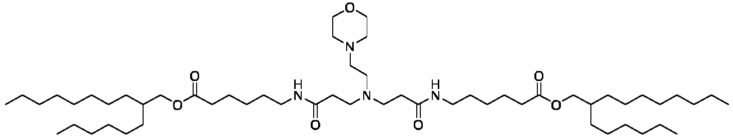 |
| T | 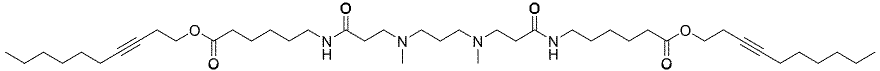 |
| U | 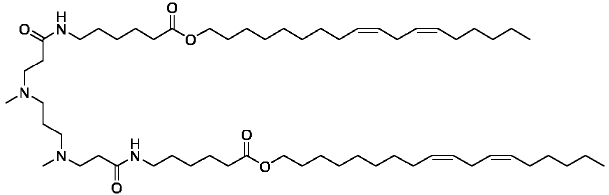 |
| V | 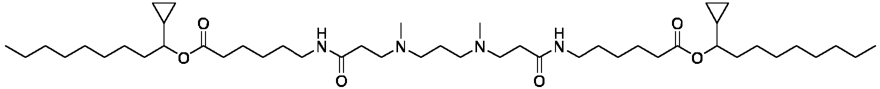 |

The second aspect of the present invention provides a method for preparing a lipid having a structure represented by formula (1-1), comprising steps of: (1) reacting a compound of formula (a) with a compound of formula (b) to obtain a compound of formula (c); (2) reacting a compound of formula (c) with a compound of formula (d) to obtain a compound of formula (e); and (3) reacting a compound of formula (e) with a compound of formula (f) and deprotecting the reaction product:

[Formula a] H₂N-(CH₂)ₐ-C(=O)OH

[Formula b] OH-R'

[Formula c] H₂N-(CH₂)ₐ-C(=O)O-R'

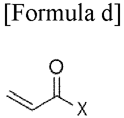

[Formula e] H₂C=CH-C(=O)-HN-(CH₂)ₐ-C(=O)O-R'

[Formula f] H₂N-(CH₂)₁₋₂₀-NH-C(=O)O-C(CH₃)₃

[Formula 1-1] H₂N-(CH₂)₁₋₂₀-N[-CH₂-CH₂-C(=O)-HN-(CH₂)ₐ-C(=O)O-R']₂

wherein,
each R' is independently 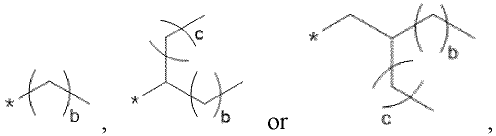 where * indicates the point of attachment to the adjacent oxygen atom, and 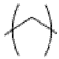 represents substituted or unsubstituted methylene group,
each of a, b and c is independently an integer of from 2 to 20, and
X is selected from the group consisting of F, CI, Br and I.

In an embodiment of the method for preparing a lipid according to the second aspect of the present invention, the reaction of step (1) may be conducted in a solvent (e.g., cyclohexane) in the presence of a catalyst (e.g., p-toluenesulfonic acid monohydrate (p-TsOH)) under reflux, the reaction of step (2) may be conducted in a solvent (e.g., methylene chloride (MC)) in the presence of a catalyst (e.g., triethylamine (TEA)) at a low temperature (e.g., from -10°C to 10°C) or room temperature (e.g., from 20°C to 30°C) condition, the reaction of step (3) may be conducted in a solvent (e.g., n-butanol (n-BuOH)) under reflux, and the deprotection of step (3) may be conducted in a solvent (e.g., methylene chloride (MC)) in the presence of an acid (e.g., trifluoroacetic acid (TFA)) at room temperature (e.g., from 20°C to 30°C) condition, but it is not limited thereto.

The third aspect of the present invention provides a method for preparing a lipid having a structure represented by formula (1-2), comprising a step of reacting a compound of formula (e) obtained from the second aspect of the present invention with a compound of formula (g):

[Formula e] H₂C=CH-C(=O)-HN-(CH₂)ₐ-C(=O)O-R'

[Formula g] H₂N-(CH₂)₁₋₂₀-N(C₁₋₂₀ alkyl)₂

[Formula 1-2] (C₁₋₂₀ alkyl)₂N-(CH₂)₁₋₂₀-N[-CH₂-CH₂-C(=O)-HN-(CH₂)ₐ-C(=O)O-R'1₂

wherein,
each R' is independently 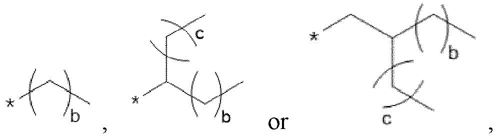 where * indicates the point of attachment to the adjacent oxygen atom, and 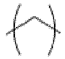 represents substituted or unsubstituted methylene group, and
each of a, b and c is independently an integer of from 2 to 20.

In an embodiment of the method for preparing a lipid according to the third aspect of the present invention, the reaction may be conducted in a solvent (e.g., n-butanol (n-BuOH)) under reflux, but it is not limited thereto.

The fourth aspect of the present invention provides a method for preparing a lipid having a structure represented by formula (1-3), comprising a step of reacting a compound of formula (e) obtained from the second aspect of the present invention with a compound of formula (h):

[Formula e] H₂C=CH-C(=O)-HN-(CH₂)ₐ-C(=O)O-R'

[Formula h] (C₁₋₁₀ alkyl)-NH-(CH₂)₁₋₂₀-NH-(C₁₋₁₀ alkyl)

[Formula 1-3] A-N(C₁₋₁₀ alkyl)-(CH₂)₁₋₂₀-N(C₁₋₁₀ alkyl)-A

wherein,
each R' is independently 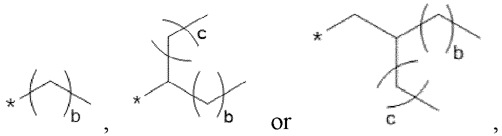 where * indicates the point of attachment to the adjacent oxygen atom, and 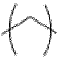 represents substituted or unsubstituted methylene group,
A is -CH₂-CH₂-C(=O)-HN-(CH₂)ₐ-C(=O)O-R',
each of a, b and c is independently an integer of from 2 to 20, and
X is selected from the group consisting of F, CI, Br and I.

In an embodiment of the method for preparing a lipid according to the fourth aspect of the present invention, the reaction may be conducted in a solvent (e.g., n-butanol (n-BuOH)) under reflux, but it is not limited thereto.

The fifth aspect of the present invention provides a method for preparing a lipid having a structure represented by formula (1-4), comprising steps of: (1) reacting a compound of formula (a) with a compound of formula (b') to obtain a compound of formula (c'); (2) reacting a compound of formula (c') with a compound of formula (d) to obtain a compound of formula (e'); and (3) reacting a compound of formula (e') with a compound of formula (h):

[Formula a] H₂N-(CH₂)ₐ-C(=O)OH

[Formula b'] OH-R₂

[Formula c'] H₂N-R₁-C(=O)O-R₂

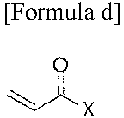

[Formula e'] H₂C=CH-C(=O)-HN-R₁-C(=O)O-R₂

[Formula h] (C₁₋₁₀ alkyl)-NH-(CH₂)₁₋₂₀-NH-(C₁₋₁₀ alkyl)

[Formula 1-4] A'-N(C₁₋₁₀ alkyl)-(CH₂)₁₋₂₀-N(C₁₋₁₀ alkyl)-A'

wherein,
A' is -CH₂-CH₂-C(=O)-HN-R₁-C(=O)O-R₂,
R₁ is independently substituted or unsubstituted, saturated or unsaturated divalent hydrocarbon group,
R₂ is independently substituted or unsubstituted, unsaturated monovalent hydrocarbon group,
a is an integer of from 2 to 20, and
X is selected from the group consisting of F, CI, Br and I.

In an embodiment of the method for preparing a lipid according to the fifth aspect of the present invention, the reaction of step (1) may be conducted in a solvent (e.g., cyclohexane) in the presence of a catalyst (e.g., p-toluenesulfonic acid monohydrate (p-TsOH)) under reflux, the reaction of step (2) may be conducted in a solvent (e.g., methylene chloride (MC)) in the presence of a catalyst (e.g., triethylamine (TEA)) at a low temperature (e.g., from -10°C to 10°C) or room temperature (e.g., from 20°C to 30°C) condition, and the reaction of step (3) may be conducted in a solvent (e.g., n-butanol (n-BuOH)) under reflux, but it is not limited thereto.

The lipid having a specific structure according to the present invention can easily form a complex with anionic drug and thus is useful for drug delivery.

Thus, the sixth aspect of the present invention provides a composition for drug delivery comprising the lipid of the present invention.

In an embodiment, the drug may be selected from nucleic acid, polypeptide, virus or combination thereof.

The "nucleic acid" may be, for example, DNA, RNA, siRNA, shRNA, miRNA, mRNA, aptamer, antisense oligonucleotide, or a combination thereof, but it is not limited thereto.

The "polypeptide" may mean a protein having activity in the body such as antibody or fragment thereof, cytokine, hormone or analog thereof, or a protein that can be recognized as antigen through a series of processes in the body, including polypeptide sequence of antigen, analog or precursor thereof.

In an embodiment, the lipid of the present invention may form a complex with the drug, and the complex is encapsulated within nanoparticle structure formed by an amphiphilic block copolymer.

In an embodiment, the amphiphilic block copolymer may be an A-B type block copolymer comprising a hydrophilic A block and a hydrophobic B block. In an aqueous environment, the A-B type block copolymer forms core-shell type polymer nanoparticle wherein the hydrophobic B block forms the core (inner wall) and the hydrophilic A block forms the shell (outer wall).

In an embodiment, the hydrophilic A block may be one or more selected from the group consisting of polyalkylene glycol, polyvinyl alcohol, polyvinyl pyrrolidone, polyacrylamide, and derivatives thereof.

More concretely, the hydrophilic A block may be one or more selected from the group consisting of monomethoxypolyethylene glycol (mPEG), monoacetoxypolyethylene glycol, polyethylene glycol, copolymer of polyethylene and propylene glycol, and polyvinylpyrrolidone.

Also, if necessary, the end of the hydrophilic A block may be chemically combined with a functional group or ligand capable of reaching specific tissue or cell, or a functional group capable of promoting intracellular delivery, in order to control *in vivo* distribution of polymer nanoparticle carrier formed by the amphiphilic block copolymer and salt of polylactic acid or to increase the efficiency of delivering the nanoparticle carrier into cell. In an embodiment, the functional group or ligand may be one or more selected from the group consisting of monosaccharides, polysaccharides, vitamins, peptides, proteins, and antibodies to cell surface receptors. More concretely, the functional group or ligand may be one or more selected from the group consisting of anisamide, vitamin B9 (folic acid), vitamin B12, vitamin A, galactose, lactose, mannose, hyaluronic acid, RGD peptide, NGR peptide, transferrin, antibody to transferrin receptor, etc.

The hydrophobic B block is a biocompatible, biodegradable polymer, and in an embodiment, it may be one or more selected from the group consisting of polyester, polyanhydride, polyamino acid, polyorthoester and polyphosphazine.

More concretely, the hydrophobic B block may be one or more selected from the group consisting of polylactide (PLA), polyglycolide, polycaprolactone, polydioxan-2-one, copolymer of polylactide and glycolide, copolymer of polylactide and polydioxan-2-one, copolymer of polylactide and and polycaprolactone, and a copolymer of polyglycolide and polycaprolactone.

Also, in an embodiment, in order to increase the hydrophobicity of the hydrophobic B block and thereby improve the stability of the nanoparticle, the hydrophobic B block may be modified by chemically combining the hydroxyl group at the end of the hydrophobic B block with tocopherol, cholesterol, or fatty acid having 10 to 24 carbons.

The present invention will be explained below in more detail with reference to the following Examples. However, the Examples are only to illustrate the invention, and the scope of the present invention is not limited thereby in any manner.

### EXAMPLES

### Example 1

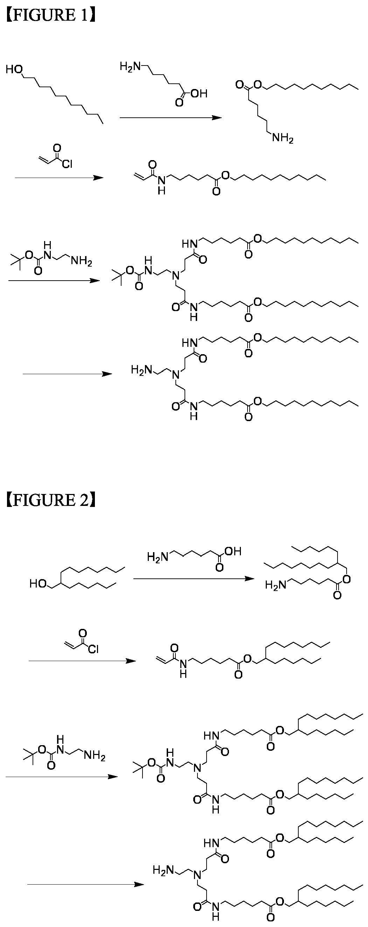

### 1-1. The compound of the following formula A was prepared according to the synthesis scheme shown in Figure 1.

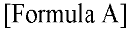

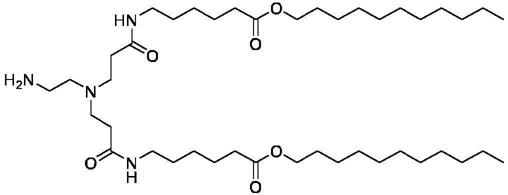

### 1-2. Synthesis of undecyl 6-acrylamidohexanoate

In a 250 mL 3-neck round bottom flask (RBF), 6-aminohexanoic acid (10 g, 76.23 mmol, 1.1 eq), undecan-1-ol (11.94 g, 69.30 mmol, 1 eq), p-toluenesulfonic acid monohydrate (p-TsOH) (15.82 g, 83.16 mmol, 1.2 eq), and cyclohexane (120 mL) were added, and a Dean-Stark trap and a condenser were installed and then stirring and reflux were performed. After 24 hours, the reaction product was concentrated in vacuo, extracted with methylene chloride (MC) and 3% sodium hydroxide aqueous solution, and the methylene chloride layer was dried over sodium sulfate and filtered. The filtrate was concentrated under vacuum to obtain undecyl 6-aminohexanoate with low purity. Without further purification, previously obtained undecyl 6-aminohexanoate, methylene chloride (100 mL), and triethylamine (TEA) (15.43 g, 152.46 mmol, 2.2 eq) were added to a 250 mL 3-neck RBF, cooled to 0°C, and acryloyl chloride (6.90 g, 76.23 mmol, 1.1 eq) was added dropwise. The temperature of the reactor was raised to room temperature (20-25°C) and stirring was performed. After 4 hours, the mixture in the reactor was extracted with a saturated aqueous solution of sodium bicarbonate, and the methylene chloride layer was dried over sodium sulfate and filtered. The filtrate was concentrated in vacuo and purified using a silica column with ethyl acetate:hexane (1:1) to obtain undecyl 6-acrylamidohexanoate (17.22 g, yield: 73%).
¹H-NMR (400 MHz, CDCl₃) *δ* 6.25 (dd, 1H), 6.01-6.08 (m, 1H), 5.61-5.68 (m, 2H), 4.01 (t, 2H), 3.31 (q, 2H), 2.28 (t, 2H), 1.43-1.68 (m, 6H), 1.30-1.45 (m, 18H), 0.89 (t, 3H)

### 1-3. Synthesis of undecyl 2,2-dimethyl-4,11-dioxo-8-(3-oxo-3-((6-oxo-6-(undecyloxy)hexyl)amino)propyl)-3-oxa-5,8,12-triazaoctadecan-18-oate

In a 100 mL 1-neck RBF, undecyl 6-acrylamidohexanoate (3 g, 8.83 mmol, 3 eq), tert-butyl (2-aminoethyl)carbamate (0.47 g, 2.94 mmol, 1 eq), and n-butanol (n-BuOH) (40 mL) were added, and then stirring and reflux were performed. After 4 days, the mixture was concentrated in vacuo at 70°C and purified using a silica column with methylene chloride: methanol: ammonium hydroxide (15:1:0.1) to obtain undecyl 2,2-dimethyl-4,11-dioxo-8-(3-oxo-3-((6-oxo-6-(undecyloxy)hexyl)amino)propyl)-3-oxa-5,8,12-triazaoctadecan-18-oate (1.34 g, yield: 54%).
¹H-NMR (400 MHz, CDCl₃) *δ* 4.06 (t, 4H), 3.49 (q, 4H), 3.26-3.16 (br, 2H), 2.71 (q, 4H), 2.48-2.41 (br, 2H), 2.32-2.85 (m, 8H), 1.67-1.26 (m, 48H), 1.44 (s, 9H), 0.87 (t, 6H)

### 1-4. Synthesis of the compound of formula A

In a 100 mL 1-neck RBF, undecyl 2,2-dimethyl-4,11-dioxo-8-(3-oxo-3-((6-oxo-6-(undecyloxy)hexyl)amino)propyl)-3-oxa-5,8,12- triazaoctadecan-18-oate (1 g, 1.19 mmol) and methylene chloride (20 mL) were added, and trifluoroacetic acid (TFA) (2 mL) was added dropwise. After stirring at room temperature for 4 hours, the mixture in the reactor was extracted with a saturated aqueous solution of sodium bicarbonate, and the methylene chloride layer was dried over sodium sulfate and filtered. The filtrate was concentrated in vacuo and purified using a silica column with methylene chloride:methanol:ammonium hydroxide (7:1:0.1) to obtain the compound of formula A (0.31 g, 35%).
¹H-NMR (400 MHz, CDCl₃) *δ* 6.86 (t, 2H), 4.06 (t, 4H), 3.25 (t, 4H), 2.89 (t, 2H), 2.68 (t, 4H), 2.58 (t, 2H), 2.36 (t, 4H), 2.31 (t, 2H), 1.65 - 1.26 (m, 48H), 0.89 (t, 6H)

### Example 2

### 2-1. The compound of the following formula B was prepared according to the synthesis scheme shown in Figure 2.

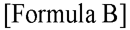

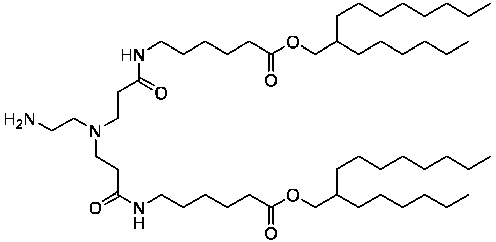

### 2-2. Synthesis of 2-hexyldecyl 6-acrylamidohexanoate

In a 250 mL 3-neck RBF, 6-aminohexanoic acid (10 g, 76.23 mmol, 1.1 eq), 2-hexyldecan-1-ol (16.80 g, 69.30 mmol, 1 eq), p-toluenesulfonic acid monohydrate (p-TsOH) (15.82 g, 83.16 mmol, 1.2 eq), and cyclohexane (120 mL) were added, and a Dean-Stark trap and a condenser were installed and then stirring and reflux were performed. After 24 hours, the mixture was cooled to room temperature, concentrated in vacuo, extracted with methylene chloride and 3% aqueous sodium hydroxide solution, and the methylene chloride layer was dried over sodium sulfate and filtered. The filtrate was concentrated in vacuo to obtain 2-hexyldecyl 6-aminohexanoate with low purity. Without further purification, previously obtained 2-hexyldecyl 6-aminohexanoate, methylene chloride (100 mL), and triethylamine (TEA) (15.43 g, 152.46 mmol, 2.2 eq) were added to a 250 mL 3-neck RBF, cooled to 0°C, and acryloyl chloride (6.90 g, 76.23 mmol, 1.1 eq) was added dropwise. The temperature of the reactor was raised to room temperature (20-25°C) and stirring was performed. After 4 hours, the mixture in the reactor was extracted with a saturated aqueous solution of sodium bicarbonate, and the methylene chloride layer was dried over sodium sulfate and filtered. The filtrate was concentrated in vacuo and purified using a silica column with ethyl acetate:hexane (1:1) to obtain 2-hexyldecyl 6-acrylamidohexanoate (14.25 g, yield: 50%).
¹H-NMR (400 MHz, CDCl₃) *δ* 6.27 (dd, 1H), 6.07-6.12 (m, 1H), 5.62-5.64 (m, 2H), 3.97 (d, 2H), 3.34 (q, 2H), 2.32 (t, 2H), 1.54-1.68 (m, 5H), 1.26-1.41 (m, 26H), 0.87 (t, 6H)

### 2-3. Synthesis of 2-hexyldecyl 8-(3-((6-((2-hexyldecyl)oxy)-6-oxohexyl)amino)-3-oxopropyl)-2,2-dimethyl-4,11-dioxo-3-oxa-5,8,12-triazaoctadecan-18-oate

In a 100 mL 1-neck RBF, 2-hexyldecyl 6-acrylamidohexanoate (4 g, 9.76 mmol, 3 eq), tert-Butyl (2-aminoethyl) carbamate (0.52 g, 3.25 mmol, 1 eq), and n-butanol (n-BuOH) (40 mL) were added, and then stirring and reflux were performed. After 4 days, the mixture was concentrated in vacuo at 70°C and purified using a silica column with methylene chloride:methanol:ammonium hydroxide (15:1:0.1) to obtain 2-hexyldecyl 8-(3-((6-((2-hexyldecyl)oxy)-6-oxohexyl)amino)-3-oxopropyl)-2,2-dimethyl-4,11-dioxo-3-oxa-5,8,12-triazaoctadecan-18-oate (2.23 g, yield: 70%).

### 2-4. Synthesis of the compound of formula B

In a 100 mL 1-neck RBF, 2-hexyldecyl 8-(3-((6-((2-hexyldecyl)oxy)-6-oxohexyl)amino)-3-oxopropyl)-2,2-dimethyl-4,11-dioxo-3-oxa-5,8,12-triazaoctadecan-18-oate (2 g, 2.04 mmol) and methylene chloride (40 mL) were added, and trifluoroacetic acid (TFA) (4 mL) was added dropwise. After stirring at room temperature for 4 hours, the mixture in the reactor was extracted with a saturated aqueous solution of sodium bicarbonate, and the methylene chloride layer was dried over sodium sulfate and filtered. The filtrate was concentrated in vacuo and purified using a silica column with methylene chloride:methanol:ammonium hydroxide (7:1:0.1) to obtain the compound of formula B (1.21 g, 67%).
¹H-NMR (400 MHz, CDCl₃) δ 6.96 (t, 2H), 3.96 (t, 4H), 3.21 - 3.13 (m, 6H), 2.71 (t, 2H), 2.64 (t, 4H), 2.37 (t, 4H), 2.31 (t, 4H), 1.65 - 1.33 (m, 62H), 0.89 (t, 6H)

### Example 3

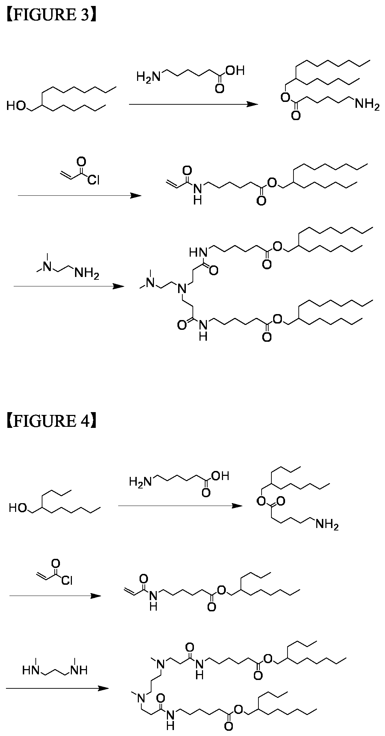

### 3-1. The compound of the following formula C was prepared according to the synthesis scheme shown in Figure 3.

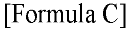

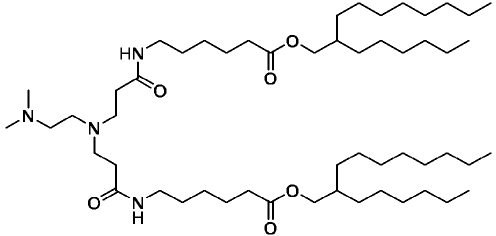

### 3-2. Synthesis of the compound of formula C

In a 100 mL 1-neck RBF, 2-hexyldecyl 6-acrylamidohexanoate (3 g, 7.32 mmol, 3 eq) synthesized in Example 2-2, N,N-dimethylethylenediamine (0.22 g, 2.44 mmol, 1 eq), and n-butanol (n-BuOH) (30 mL) were added, and then stirring and reflux were performed. After 3 days, the mixture was concentrated in vacuo at 70°C, and purified using a silica column with methylene chloride: methanol: ammonium hydroxide (10:1:0.1) to obtain the compound of formula C (0.95 g, yield: 43%).
¹H-NMR (400 MHz, CDCl₃) δ 4.05 (t, 4H), 3.15 (q, 4H), 2.77 (br, 4H), 2.69 (t, 4H), 2.53 (br, 6H), 2.12 (t, 4H), 2.28 (t, 4H), 1.66 - 1.26 (m, 62H), 0.89 (t, 6H)

### Example 4

### 4-1. The compound of the following formula D was prepared according to the synthesis scheme shown in Figure 4.

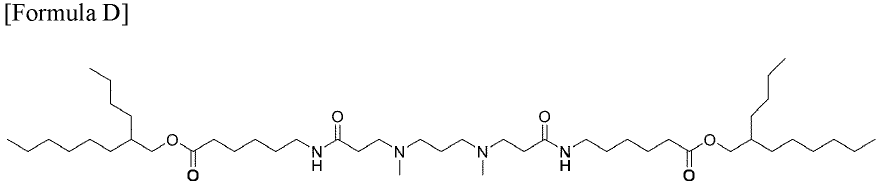

### 4-2. Synthesis of 2-butyloctyl 6-acrylamidohexanoate

In a 500 mL 3-neck RBF, 6-aminohexanoic acid (5.03 g, 38.32 mmol, 1.20 eq), 2-butyl-1-n-octanol (5.95 g, 31.93 mmol, 1.00 eq), p-toluenesulfonic acid monohydrate (10.93 g, 57.48 mmol, 1.80 eq), and cyclohexane (200 mL) were added, and a Dean-Stark trap and a condenser were installed and then stirring and reflux were performed. After 24 hours, the mixture was cooled to room temperature, concentrated in vacuo, extracted with methylene chloride and 3% aqueous sodium hydroxide solution, and the methylene chloride layer was dried over sodium sulfate and filtered. The filtrate was concentrated in vacuo to obtain 2-butyloctyl 6-aminohexanoate with low purity. Without further purification, previously obtained 2-butyloctyl 6-aminohexanoate, methylene chloride (170 mL), and triethylamine (7.11 g, 70.28 mmol, 2.20 eq) were added to a 250 mL 3-neck RBF, cooled to 0°C, and acryloyl chloride (3.18 g, 35.12 mmol, 1.10 eq) was added dropwise. The temperature of the reactor was raised to room temperature (20-25°C) and stirring was performed. After 18 hours, the mixture in the reactor was extracted with HCl aqueous solution, and the organic layer was dried over sodium sulfate and filtered. The filtrate was concentrated in vacuo and purified using a silica column with ethyl acetate (EtOAc):hexane (1:2) to obtain 2-butyloctyl 6-acrylamidohexanoate (5.0 g, yield: 44%).
¹H-NMR (400 MHz, CDCl₃) *δ* 6.47 (dd, 1H), 6.09-6.15 (m, 1H), 5.63 (dd, 2H), 3.97 (d, 2H), 3.34 (q, 2H), 2.32 (t, 2H), 1.54-1.68 (m, 6H), 1.27-1.40 (m, 20H), 0.87-0.91 (m, 6H)

### 4-3. Synthesis of the compound of formula D

In a 100 mL 3-neck RBF, 2-butyloctyl 6-acrylamidohexanoate (1000.00 mg, 2.83 mmol, 2.60 eq), N,N'-dimethyl-1,3-propanediamine (111.16 mg, 1.09 mmol, 1.00 eq), and n-BuOH (11 mL) were added, and then stirring and reflux were performed. After 24 hours, the mixture was concentrated in vacuo at 80°C, and purified using a silica column with methylene chloride:methanol:ammonium hydroxide (15:1:0.1) to obtain the compound of formula D (712.60 mg, yield: 81%).
¹H-NMR (400 MHz, CDCl₃) δ 7.87 (s, 2H), 3.96 (d, 4H), 3.22 (q, 4H), 2.61 (t, 4H), 2.41 (t, 4H), 2.36 (t, 4H), 2.30 (t, 4H), 2.25 (s, 6H), 1.61-1.70 (m, 8H), 1.48-1.54 (m, 4H), 1.25-1.39 (m, 37H), 0.90 (t, 3H)

### Example 5

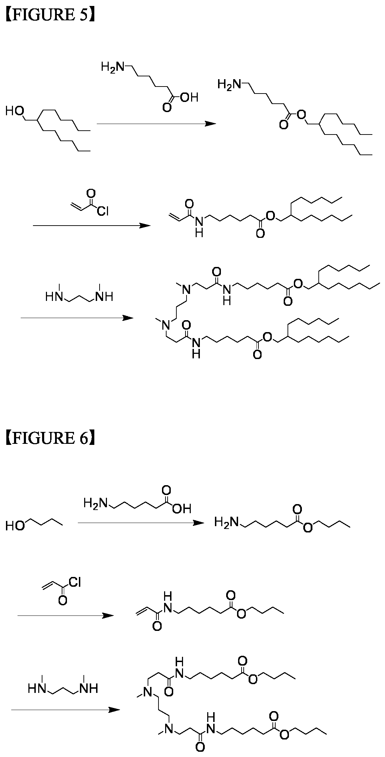

### 5-1. The compound of the following formula E was prepared according to the synthesis scheme shown in Figure 5.

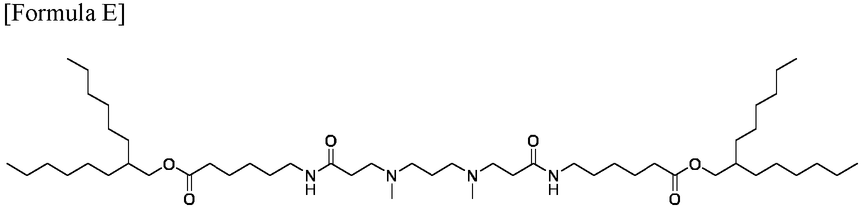

### 5-2. Synthesis of 2-hexyloctyl 6-acrylamidohexanoate

In a 500 mL 3-neck RBF, 6-aminohexanoic acid (1.84 g, 13.99 mmol, 1.20 eq), 2-hexyl-1-n-octanol (2.50 g, 11.66 mmol, 1.00 eq), p-toluenesulfonic acid monohydrate (3.99 g, 20.99 mmol, 1.80 eq), and cyclohexane (120 mL) were added, and a Dean-Stark trap and a condenser were installed and then stirring and reflux were performed. After 24 hours, the mixture was cooled to room temperature, concentrated in vacuo, extracted with methylene chloride and 3% aqueous sodium hydroxide solution, and the methylene chloride layer was dried over sodium sulfate and filtered. The filtrate was concentrated in vacuo to obtain 2-hexyloctyl 6-aminohexanoate with low purity. Without further purification, the previously obtained 2-hexyloctyl 6-aminohexanoate, methylene chloride (60 mL), triethylamine (2.60 g, 25.65 mmol, 2.20 eq), and methylene chloride (60 mL) were added to a 250 mL 3-neck RBF and cooled to 0°C, and acryloyl chloride (1.16 g, 12.83 mmol, 1.10 eq) was added dropwise. The temperature of the reactor was raised to room temperature (20-25°C) and stirring was performed. After 18 hours, the mixture in the reactor was extracted with HCl aqueous solution, and the organic layer was dried over sodium sulfate and filtered. The filtrate was concentrated in vacuo and purified using a silica column with ethyl acetate (EtOAc):hexane (1:2) to obtain 2-hexyloctyl 6-acrylamidohexanoate (3.63 g, yield: 82%).
¹H-NMR (400 MHz, CDCl₃) *δ* 6.27 (dd, 1H), 6.05-6.11 (m, 1H), 5.63 (dd, 2H), 3.97 (d, 2H), 3.34 (q, 2H), 2.32 (t, 2H), 1.41-1.68 (m, 6H), 1.21-1.41 (m, 20H), 0.85-0.90 (m, 6H)

### 5-3. Synthesis of the compound of formula E

In a 100 mL 3-neck RBF, 2-hexyloctyl 6-acrylamidohexanoate (660.27 mg, 1.73 mmol, 2.60 eq), N,N'-dimethyl-1,3-propanediamine (68.00 mg, 0.67 mmol, 1.00 eq), and n-BuOH (7 mL) were added, and then stirring and reflux were performed. After 24 hours, the mixture was concentrated in vacuo at 80°C and purified using a silica column with methylene chloride:methanol:ammonium hydroxide (15:1:0.1) to obtain the compound of formula E (375.60 mg, yield: 65%).
¹H-NMR (400 MHz, CDCl₃) δ 7.85 (s, 2H), 3.96 (d, 4H), 3.22 (q, 4H), 2.62 (t, 4H), 2.42 (t, 4H), 2.36 (t, 4H), 2.30 (t, 4H), 2.25 (s, 6H), 1.61-1.71 (m, 8H), 1.48-1.54 (m, 4H), 1.20-1.40 (m, 45H), 0.90 (t, 12H)

### Example 6

### 6-1. The compound of the following formula F was prepared according to the synthesis scheme shown in Figure 6.

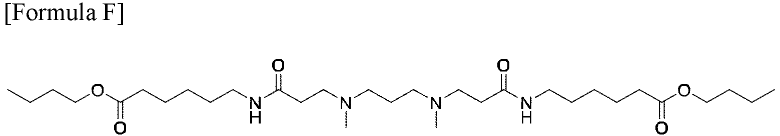

### 6-2. Synthesis of butyl 6-acrylamidohexanoate

In a 500 mL 3-neck RBF, 6-aminohexanoic acid (6.37 g, 48.57 mmol, 1.20 eq), butan-1-ol (3.00 g, 40.47 mmol, 1.00 eq), p-toluenesulfonic acid monohydrate (15.40 g, 80.95 mmol, 2.00 eq), and cyclohexane (200 mL) were added, and a Dean-Stark trap and a condenser were installed and then stirring and reflux were performed. After 24 hours, the mixture was cooled to room temperature, concentrated in vacuo, extracted with methylene chloride and 3% sodium hydroxide aqueous solution, and the methylene chloride layer was dried over sodium sulfate and filtered. The filtrate was concentrated in vacuo to obtain butyl 6-aminohexanoate with low purity. Without further purification, previously obtained butyl 6-aminohexanoate, methylene chloride (200 mL), and triethylamine (9.01 g, 89.04 mmol, 2.20 eq) were added to a 500 mL 3-neck RBF and cooled to 0°C, and acryloyl chloride (4.03 g, 44.52 mmol, 1.10 eq) was added dropwise. The temperature of the reactor was raised to room temperature (20-25 °C) and stirring was performed. After 18 hours, the mixture in the reactor was extracted with aqueous HCl solution, and the organic layer was dried over sodium sulfate and filtered. The filtrate was concentrated in vacuo and purified using a silica column with EtOAc:hexane (1:1) to obtain butyl 6-acrylamidohexanoate (3.58 g, yield: 37%).
¹H-NMR (400 MHz, CDCl₃) *δ* 6.27 (dd, 1H), 6.01-6.11 (m, 1H), 5.62-5.67 (m, 2H), 4.07 (t, 2H), 3.34 (q, 2H), 2.31 (t, 2H), 1.41-1.62 (m, 6H), 1.34-1.39 (m, 4H), 0.93 (t, 3H)

### 6-3. Synthesis of the compound of formula F

In a 100 mL 3-neck RBF, butyl 6-acrylamidohexanoate (700.00 mg, 2.90 mmol, 2.60 eq), N,N'-dimethyl-1,3-propanediamine (113.99 mg, 1.12 mmol, 1.00 eq), and n-BuOH (11 mL) were added, and then stirring and reflux were performed. After 24 hours, the mixture was concentrated in vacuo at 80°C and purified using a silica column with methylene chloride: methanol: ammonium hydroxide (15:1:0.1) to obtain the compound of formula F (479.00 mg, yield: 73%).
¹H-NMR (400 MHz, CDCl₃) *δ* 7.89 (s, 2H), 4.06 (t, 4H), 3.22 (q, 4H), 2.61 (t, 4H), 2.46 (t, 4H), 2.42 (t, 4H), 2.36 (t, 4H), 2.31 (s, 6H), 1.60-1.70 (m, 10H), 1.41-1.60 (m, 4), 1.32-1.39 (m, 8H), 0.90 (t, 6H)

### Example 7

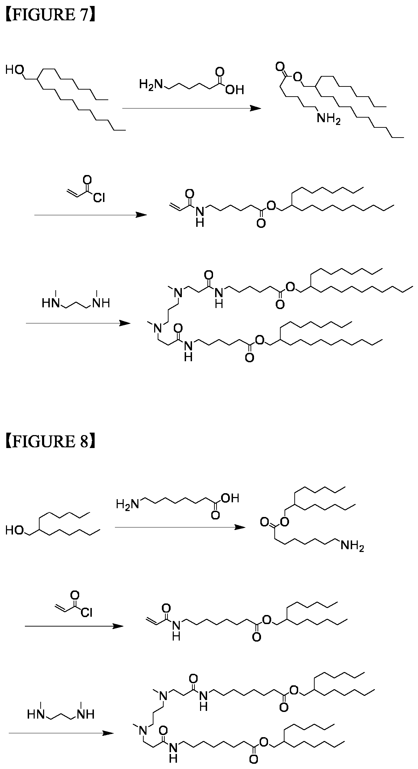

### 7-1. The compound of the following formula G was prepared according to the synthesis scheme shown in Figure 7.

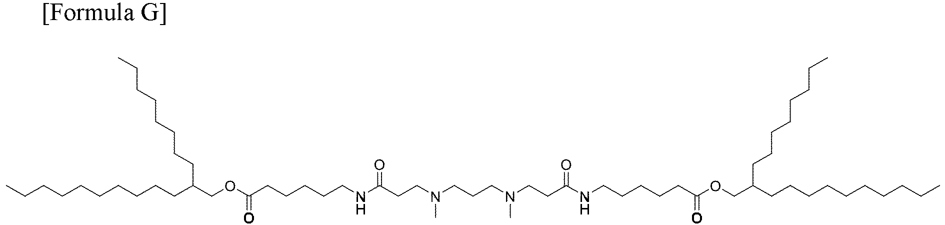

### 7-2. Synthesis of 2-octyldodecyl 6-acrylamidohexanoate

In a 250 mL 3-neck RBF, 6-aminohexanoic acid (1.05 g, 8.04 mmol, 1.20 eq), 2-octyldodecan-1-ol (2.00 g, 6.70 mmol, 1.00 eq), p-toluenesulfonic acid monohydrate (2.29 g, 12.06 mmol, 1.80 eq), and cyclohexane (100 mL) were added, and a Dean-Stark trap and a condenser were installed and then stirring and reflux were performed. After 24 hours, the mixture was cooled to room temperature, concentrated in vacuo, extracted with methylene chloride and 3% sodium hydroxide aqueous solution, and the methylene chloride layer was dried over sodium sulfate and filtered. The filtrate was concentrated in vacuo to obtain 2-octyldodecyl 6-aminohexanoate with low purity. Without further purification, previously obtained 2-octyldodecyl 6-aminohexanoate, methylene chloride (33 mL), and triethylamine (1.49 g, 14.74 mmol, 2.20 eq) were added to a 100 mL 3-neck RBF and cooled to 0°C, and acryloyl chloride (0.67 g, 7.37 mmol, 1.10 eq) was added dropwise. The temperature of the reactor was raised to room temperature (20-25°C) and stirring was performed. After 18 hours, the mixture in the reactor was extracted with aqueous HCl solution, and the organic layer was dried over sodium sulfate and filtered. The filtrate was concentrated in vacuo and purified using a silica column with EtOAc:hexane (1:2) to obtain 2-octyldodecyl 6-acrylamidohexanoate (1928.20 mg, yield: 62%).
¹H-NMR (400 MHz, CDCl₃) *δ* 6.27 (dd, 1H), 6.05-6.10 (m, 1H), 5.59-5.64 (m, 2H), 3.97 (d, 2H), 3.34 (q, 2H), 2.32 (t, 2H), 1.54-1.68 (m, 4H), 1.26-1.41 (m, 36H), 0.89 (t, 6H)

### 7-3. Synthesis of the compound of formula G

In a 100 mL 3-neck RBF, 2-octyldodecyl 6-acrylamidohexanoate (592.57 mg, 1.27 mmol, 2.60 eq), N,N'-dimethyl-1,3-propanediamine (50.00 mg, 0.49 mmol, 1.00 eq), and n-BuOH (5 mL) were added, and then stirring and reflux were performed. After 24 hours, the mixture was concentrated in vacuo at 80°C and purified using a silica column with methylene chloride:methanol:ammonium hydroxide (10:1:0.1) to obtain the compound of formula G (346.8 mg, yield: 69%).
¹H-NMR (400 MHz, CDCl₃) δ 7.83 (s, 2H), 3.96 (d, 4H), 3.22 (q, 4H), 2.62 (t, 4H), 2.42 (t, 4H), 2.36 (t, 4H), 2.30 (t, 4H), 2.25 (s, 6H), 1.61-1.69 (m, 8H), 1.48-1.54 (m, 4H), 1.25-1.39 (m, 69H), 0.90 (t, 3H)

### Example 8

### 8-1. The compound of the following formula H was prepared according to the synthesis scheme shown in Figure 8.

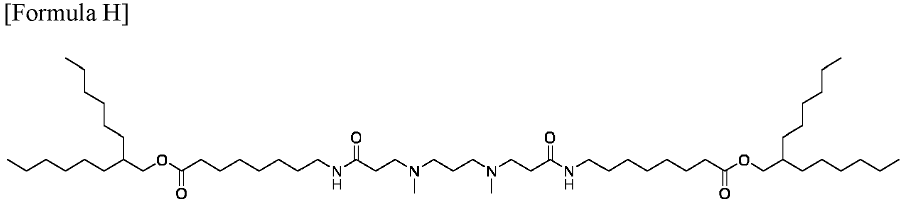

### 8-2. Synthesis of 2-hexyloctyl 8-acrylamidooctanoate

In a 250 mL 3-neck RBF, 8-aminoctanoic acid (1.78 g, 11.19 mmol, 1.20 eq), 2-hexyloctan-1-ol (2.00 g, 9.33 mmol, 1.00 eq), p-toluenesulfonic acid monohydrate (3.19 g, 16.79 mmol, 1.80 eq), and cyclohexane (100 mL) were added, and a Dean-Stark trap and a condenser were installed and then stirring and reflux were performed. After 24 hours, the mixture was cooled to room temperature, concentrated in vacuo, extracted with methylene chloride and 3% sodium hydroxide aqueous solution, and the methylene chloride layer was dried over sodium sulfate and filtered. The filtrate was concentrated in vacuo to obtain 2-hexyloctyl 8-aminooctanoate with low purity. Without further purification, previously obtained 2-hexyloctyl 8-aminooctanoate, methylene chloride (100 mL), and triethylamine (2.08 g, 20.52 mmol, 2.20 eq) were added to a 250 mL 3-neck RBF and cooled to 0°C, and acryloyl chloride (0.93 g, 10.26 mmol, 1.10 eq) was added dropwise. The temperature of the reactor was raised to room temperature (20-25°C) and stirring was performed. After 18 hours, the mixture in the reactor was extracted with aqueous HCl solution, and the organic layer was dried over sodium sulfate and filtered. The filtrate was concentrated in vacuo and purified using a silica column with EtOAc:hexane (1:1) to obtain 2-hexyloctyl 8-acrylamidooctanoate (2838.70 mg, yield: 74%).
¹H-NMR (400 MHz, CDCl₃) *δ* 6.27 (dd, 1H), 6.05-6.10 (m, 1H), 5.55-5.64 (m, 2H), 3.97 (d, 2H), 3.32 (q, 2H), 2.29 (t, 2H), 1.51-1.65 (m, 4H), 1.27-1.35 (m, 27H), 0.85-0.90 (m, 6H)

### 8-3. Synthesis of the compound of formula H

In a 100 mL 3-neck RBF, 2-hexyloctyl 8-acrylamidooctanoate (703.42 mg, 0.76 mmol, 2.60 eq), N,N'-dimethyl-1,3-propanediamine (30.00 mg, 0.29 mmol, 1.00 eq), and n-BuOH (5 mL) were added, and then stirring and reflux were performed. After 24 hours, the mixture was concentrated in vacuo at 80°C and purified using a silica column with methylene chloride:methanol:ammonium hydroxide (10:1:0.1) to obtain the compound of formula H (218.90 mg, yield: 81%).
¹H-NMR (400 MHz, CDCl₃) δ 7.78 (s, 2H), 3.96 (d, 4H), 3.21 (q, 4H), 2.64 (t, 4H), 2.44 (t, 4H), 2.38 (t, 4H), 2.28-2.32 (m, 4H), 2.26 (s, 6H), 1.66-1.72 (m, 2H), 1.40-1.63 (m, 10H), 1.20-1.40 (m, 54H), 0.90 (t, 12H)

### Example 9

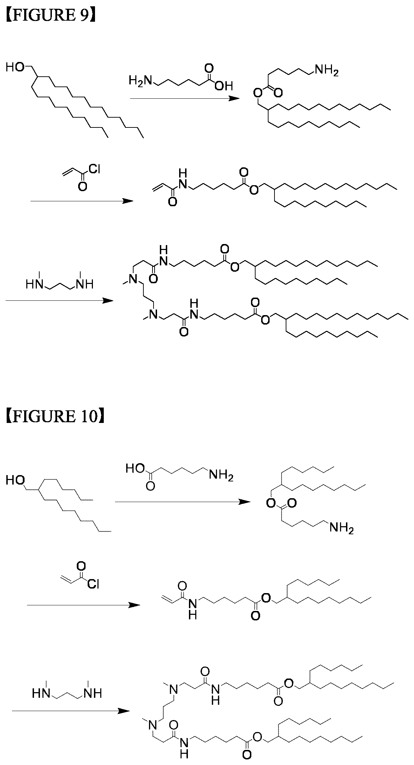

### 9-1. The compound of the following formula I was prepared according to the synthesis scheme shown in Figure 9.

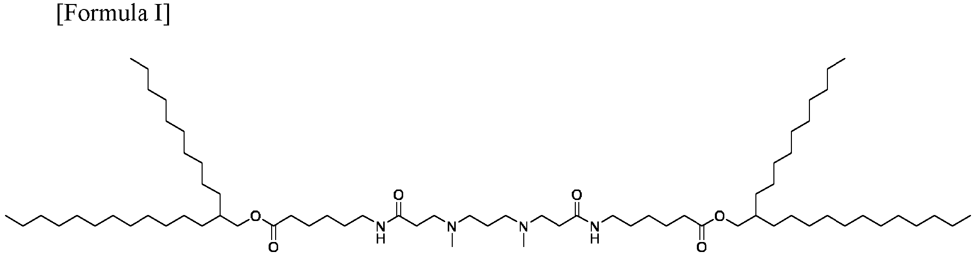

### 9-2. Synthesis of 2-decyltetradecyl 6-acrylamidohexanoate

In a 250 mL 3-neck RBF, 6-aminohexanoic acid (887.70 mg, 6.77 mmol, 1.20 eq), 2-decyltetradecan-1-ol (2.00 g, 5.64 mmol, 1.00 eq), p-toluenesulfonic acid monohydrate (1.93 g, 10.15 mmol, 1.80 eq), and cyclohexane (100 mL) were added, and a Dean-Stark trap and a condenser were installed and then stirring and reflux were performed. After 24 hours, the mixture was cooled to room temperature, concentrated in vacuo, extracted with methylene chloride and 3% sodium hydroxide aqueous solution, and the methylene chloride layer was dried over sodium sulfate and filtered. The filtrate was concentrated in vacuo to obtain 2-decyltetradecyl 6-aminohexanoate with low purity. Without further purification, previously obtained 2-decyltetradecyl 6-aminohexanoate, methylene chloride (100 mL), and triethylamine (1.26 g, 12.41 mmol, 2.20 eq) were added to a 250 mL 3-neck RBF and cooled to 0°C, and acryloyl chloride (561.44 mg, 6.20 mmol, 1.10 eq) was added dropwise. The temperature of the reactor was raised to room temperature (20-25°C) and stirring was performed. After 18 hours, the mixture in the reactor was extracted with aqueous HCl solution, and the organic layer was dried over sodium sulfate and filtered. The filtrate was concentrated in vacuo and purified using a silica column with EtOAc:hexane (1:2) to obtain 2-decyltetradecyl 6-acrylamidohexanoate (1.29 g, yield: 44%).
¹H-NMR (400 MHz, CDCl₃) *δ* 6.27 (dd, 1H), 6.05-6.10 (m, 1H), 5.62-5.64 (m, 2H), 3.97 (d, 2H), 3.34 (q, 2H) 2.31 (t, 2H), 1.54-1.68 (m, 5H), 1.26-1.41 (m, 44H), 0.88-0.89 (m, 6H)

### 9-3. Synthesis of the compound of formula I

In a 100 mL 3-neck RBF, 2-decyltetradecyl 6-acrylamidohexanoate (306.44 mg, 0.59 mmol, 2.40 eq), N,N'-dimethyl-1,3-propanediamine (25.00 mg, 0.24 mmol, 1.00 eq), and n-BuOH (2.5 mL) were added, and then stirring and reflux were performed. After 24 hours, the mixture was concentrated in vacuo at 80°C and purified using a silica column with methylene chloride:methanol:ammonium hydroxide (10:1:0.1) to obtain the compound of formula I (209.2 mg, yield: 75%).
¹H-NMR (400 MHz, CDCl₃) δ 7.86 (s, 2H), 3.96 (d, 4H), 3.22 (q, 4H), 2.60 (t, 4H), 2.41 (t, 4H), 2.35 (t, 4H), 2.30 (t, 4H), 2.24 (s, 6H), 1.61-1.69 (m, 11H), 1.48-1.53 (m, 4H), 1.20-1.40 (m, 87H), 0.9 (t, 12H)

### Example 10

### 10-1. The compound of the following formula J was prepared according to the synthesis scheme shown in Figure 10.

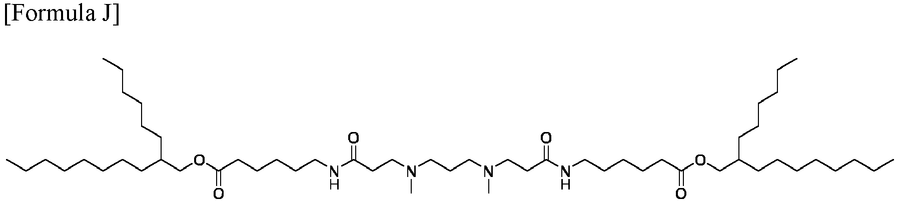

### 10-2. Synthesis of the compound of formula J

In a 100 mL 3-neck RBF, 2-hexyldecyl 6-acrylamidohexanoate (2000.00 mg, 4.88 mmol, 3.00 eq) synthesized in Example 2-2, N,N'-dimethyl-1,3-propanediamine (170.00 mg, 1.63 mmol, 1.00 eq), and n-BuOH (20 mL) were added, and then stirring and reflux were performed. After 24 hours, the mixture was concentrated in vacuo at 80°C and purified using a silica column with methylene chloride: methanol: ammonium hydroxide (10:1:0.1) to obtain the compound of chemical formula J (934.00 mg, yield: 62%).
¹H-NMR (400 MHz, CDCl₃) δ 7.88 (s, 2H), 3.96 (d, 4H), 3.22 (q, 4H), 2.60 (t, 4H), 2.40 (t, 4H), 2.35 (t, 4H), 2.30 (t, 4H), 2.27 (s, 6H), 1.61-1.70 (m, 8H), 1.40-1.54 (m, 4H), 1.20-1.40 (m, 52H), 0.90 (t, 12H)

### Example 11

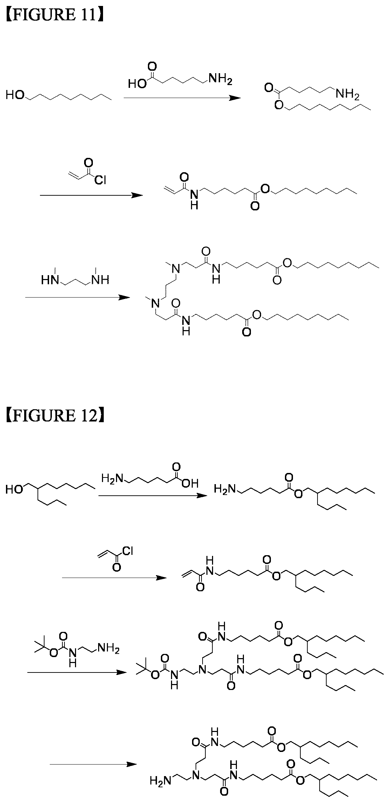

### 11-1. The compound of the following formula K was prepared according to the synthesis scheme shown in Figure 11.

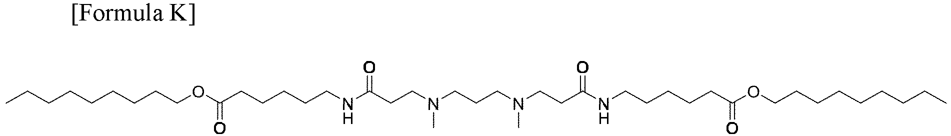

### 11-2. Synthesis of nonyl 6-acrylamidohexanoate

In a 250 mL 3-neck RBF, 6-aminohexanoic acid (10 g, 76.23 mmol, 1.1 eq), nonan-1-ol (21.58 g, 69.30 mmol, 1 eq), p-toluenesulfonic acid monohydrate (p-TsOH) (15.82 g, 83.16 mmol, 1.2 eq), and cyclohexane (120 mL) were added, and a Dean-Stark trap and a condenser were installed and then stirring and reflux were performed. After 24 hours, the mixture was concentrated in vacuo, extracted with methylene chloride and 3% aqueous sodium hydroxide solution, and the methylene chloride layer was dried over sodium sulfate and filtered. The filtrate was concentrated in vacuo to obtain nonyl 6-aminohexanoate with low purity. Without further purification, previously obtained nonyl 6-aminohexanoate, methylene chloride (100 mL), and triethylamine (15.43 g, 152.46 mmol, 2.2 eq) were added to a 250 mL 3-neck RBF and cooled to 0°C, and acryloyl chloride (6.90 g, 76.23 mmol, 1.1 eq) was added dropwise. The temperature of the reactor was raised to room temperature (20-25°C) and stirring was performed. After 4 hours, the mixture in the reactor was extracted with a saturated aqueous sodium bicarbonate solution, and the methylene chloride layer was dried over sodium sulfate and filtered. The filtrate was concentrated in vacuo and purified using a silica column with EtOAc:hexane (1:1) to obtain nonyl 6-acrylamidohexanoate (15.66 g, yield: 66%).
¹H-NMR (400 MHz, CDCl₃) *δ* 6.28 (d, 1H), 6.11 (m, 1H), 5.64 (d, 2H), 4.07 (t, 2H), 3.63 (q, 2H), 2.32 (t, 2H), 1.68 - 1.26 (m, 20H), 0.90 (t, 3H)

### 11-3. Synthesis of the compound of formula K

In a 100 mL 3-neck RBF, nonyl 6-acrylamidohexanoate (1000.00 mg, 3.21 mmol, 2.60 eq), N,N'-dimethyl-1,3-propanediamine (126.18 mg, 1.23 mmol, 1.00 eq), and n-BuOH (10 mL) were added, and then stirring and reflux were performed. After 24 hours, the mixture was concentrated in vacuo at 80°C and purified using a silica column with methylene chloride:methanol:ammonium hydroxide (7:1:0.1) to obtain the compound of formula K (770.10 mg, yield: 86%).
¹H-NMR (400 MHz, CDCl₃) *δ* 7.87 (s, 2H), 4.05 (t, 4H), 3.22 (q, 4H), 2.62 (t, 4H), 2.42 (t, 4H), 2.36 (t, 2H), 2.30 (t, 4H), 2.25 (s, 6H), 1.61-1.69 (m, 10H), 1.40-1.60 (m, 4H), 1.27-1.38 (m, 28H), 0.87 (t, 6H)

### Example 12

### 12-1. The compound of the following formula L was prepared according to the synthesis scheme shown in Figure 12.

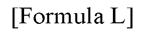

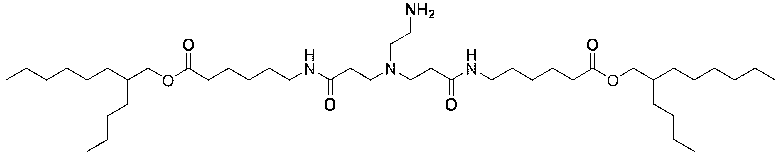

### 12-2. Synthesis of the compound of formula L

In a 100 mL 3-neck RBF, 2-butyloctyl 6-acrylamidohexanoate (850.65 mg, 2.43 mmol, 2.60 eq) synthesized in Example 4-2, tert-butyl N-(2-aminoethyl)carbamate (150.00 mg, 0.94 mmol, 1.00 eq), and n-BuOH (10 mL) were added, and then stirring and reflux were performed. After 48 hours, the mixture was concentrated in vacuo at 80°C and purified using a silica column with methylene chloride:methanol:ammonium hydroxide (15:1:0.1) to obtain 2-butyloctyl 8-(3-((6-((2-butyloctyl)oxy)-6-oxohexyl)amino)-3-oxopropyl)-2,2-dimethyl-4,11-dioxo-3-oxa-5,8,12-triazaoctadecan-18-oate with low purity. Subsequently, the synthesis of the compound of formula L was conducted without further purification process. In a 25 mL 1-neck RBF, 2-butyloctyl 8-(3-((6-((2-butyloctyl)oxy)-6-oxohexyl)amino)-3-oxopropyl)-2,2-dimethyl-4,11-dioxo-3-oxa-5,8,12-triazaoctadecan-18-oate (143.20 mg, 0.16 mmol) and methylene chloride (2 mL) were added, and trifluoroacetic acid (0.2 mL) was added dropwise. After stirring at room temperature for 24 hours, the mixture in the reactor was extracted with a saturated aqueous solution of sodium bicarbonate, and the organic layer was dried over sodium sulfate and filtered. The filtrate was concentrated in vacuo and purified using a silica column with methylene chloride:methanol:ammonium hydroxide (7:1:0.1) to obtain the compound of formula L (85.1 mg, 67%).
¹H-NMR (400 MHz, CDCl₃) δ 6.38 (t, 2H), 3.96 (d, 4H), 3.18 (q, 4H), 2.93 (t, 2H), 2.67 (t, 4H), 2.58 (t, 2H), 2.29-2.36 (m, 8H), 1.61-1.66 (m, 6H), 1.48-1.60 (m, 4H), 1.27-1.38 (m, 38H), 0.90-0.92 (m, 12H)

### Example 13

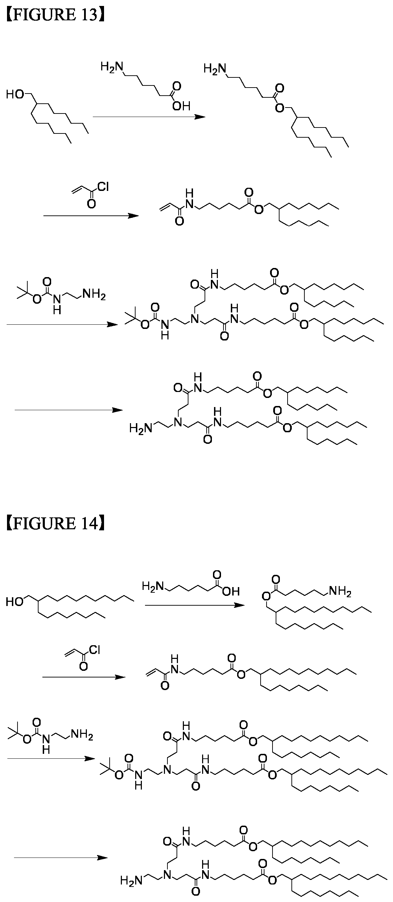

### 13-1. The compound of the following formula M was prepared according to the synthesis scheme shown in Figure 13.

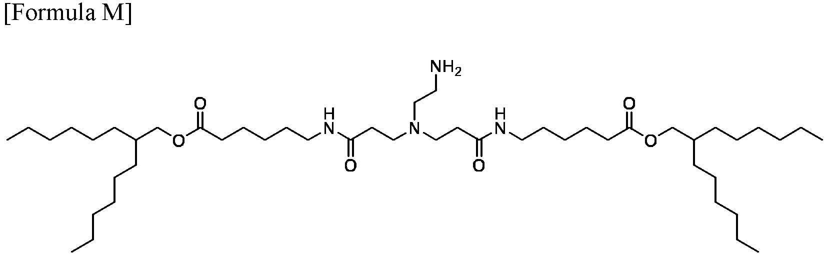

### 13-2. Synthesis of the compound of formula M

In a 100 mL 3-neck RBF, 2-hexyloctyl 6-acrylamidohexanoate (1429.07 mg, 3.74 mmol, 2.40 eq) synthesized in Example 5-2, tert-butyl N-(2-aminoethyl)carbamate (250.00 mg, 1.56 mmol, 1.00 eq), and n-BuOH (10 mL) were added, and then stirring and reflux were performed. After 48 hours, the mixture was concentrated in vacuo at 80°C and purified using a silica column with methylene chloride:methanol:ammonium hydroxide (15:1:0.1) to obtain 2-hexyloctyl 8-(3-((6-((2-hexyloctyl)oxy)-6-oxohexyl)amino)-3-oxopropyl)-2,2-dimethyl-4,11-dioxo-3-oxa-5,8,12-triazaoctadecan-18-oate with low purity. Subsequently, the synthesis of the compound of formula M was conducted without further purification process. In a 25 mL 1-neck RBF, 2-hexyloctyl 8-(3-((6-((2-hexyloctyl)oxy)-6-oxohexyl)amino)-3-oxopropyl)-2,2-dimethyl-4,11-dioxo-3-oxa-5 ,8,12-triazaoctadecan-18-oate and methylene chloride (3 mL) were added, and trifluoroacetic acid (0.3 mL) was added dropwise. After stirring at room temperature for 24 hours, the mixture in the reactor was extracted with a saturated aqueous solution of sodium bicarbonate, and the organic layer was dried over sodium sulfate and filtered. The filtrate was concentrated in vacuo and purified using a silica column with methylene chloride:methanol:ammonium hydroxide (8:1:0.1) to obtain the compound of formula M (116.6 mg, two steps 9.1%).
¹H-NMR (400 MHz, CDCl₃) δ 6.94 (t, 2H), 3.96 (d, 4H), 3.12-3.20 (m, 6H), 2.73 (t, 2H), 2.62 (t, 4H), 2.29-2.37 (m, 8H), 1.59-1.65 (m, 6H), 1.47-1.52 (m, 4H), 1.20-1.39 (m, 46H), 0.90 (t, 12H)

### Example 14

### 14-1. The compound of the following formula N was prepared according to the synthesis scheme shown in Figure 14.

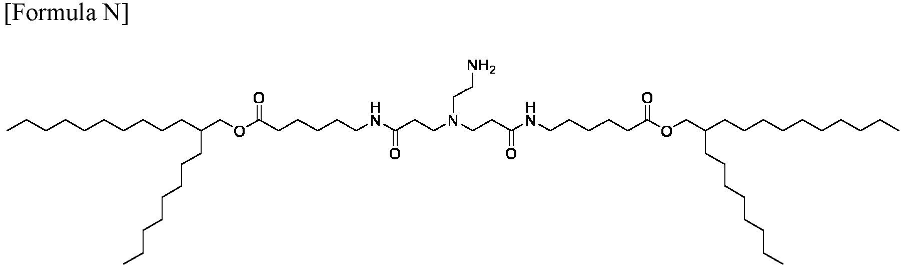

### 14-2. Synthesis of the compound of formula N

In a 100 mL 3-neck RBF, 2-octyldodecyl 6-acrylamidohexanoate (1200.00 mg, 2.58 mmol, 2.40 eq) synthesized in Example 7-2, tert-butyl N-(2-aminoethyl)carbamate (171.99 mg, 1.07 mmol, 1.00 eq), and n-BuOH (10 mL) were added, and then stirring and reflux were performed. After 48 hours, the mixture was concentrated in vacuo at 80°C and purified using a silica column with methylene chloride:methanol:ammonium hydroxide (15:1:0.1) to obtain 2-octyldodecyl 2,2-dimethyl-8-(3-((6-((2-octyldodecyl)oxy)-6-oxohexyl)amino)-3-oxopropyl)-4,11-dioxo-3-oxa-5,8,12-triazaoctadecan-18-oate with low purity. Subsequently, the synthesis of the compound of formula N was conducted without further purification process. In a 100 mL 1-neck RBF, 2-octyldodecyl 2,2-dimethyl-8-(3-((6-((2-octyldodecyl)oxy)-6-oxohexyl)amino)-3-oxopropyl)-4,11-dioxo-3-oxa-5,8,12-triazaoctadecan-18-oate and methylene chloride (10 mL) were added, and trifluoroacetic acid (1.0 mL) was added dropwise. After stirring at room temperature for 24 hours, the mixture in the reactor was extracted with a saturated aqueous solution of sodium bicarbonate, and the organic layer was dried over sodium sulfate and filtered. The filtrate was concentrated in vacuo and purified using a silica column with methylene chloride:methanol:ammonium hydroxide (7:1:0.1) to obtain the compound of formula N (137.8 mg, two steps 12.9%).
¹H-NMR (400 MHz, CDCl₃) δ 6.86 (t, 2H), 3.96 (d, 4H), 3.17 (q, 4H), 2.98 (t, 2H), 2.61-2.67 (m, 6H), 2.29-2.37 (m, 8H), 1.60-1.66 (m, 7H), 1.20-1.38 (m, 72H), 0.90-0.95 (m, 12H)

### Example 15

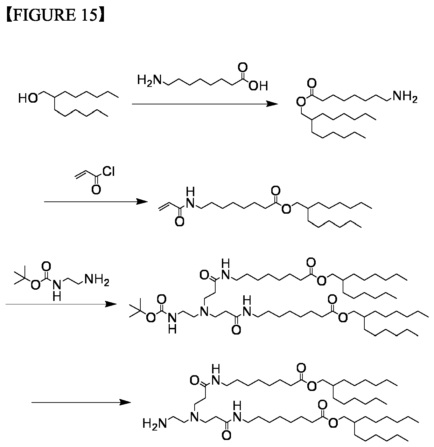

### 15-1. The compound of the following formula O was prepared according to the synthesis scheme shown in Figure 15.

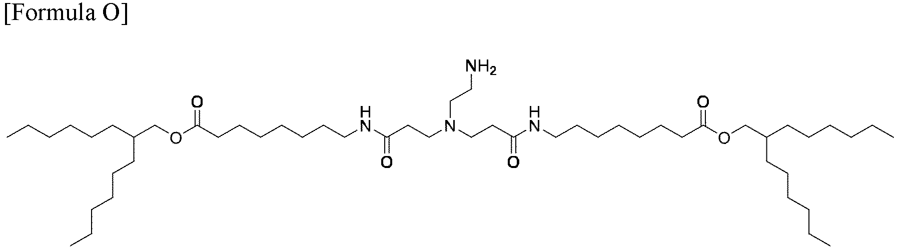

### 15-2. Synthesis of the compound of formula O

In a 100 mL 3-neck RBF, 2-hexyloctyl 8-acrylamidooctanoate (1130.14 mg, 2.76 mmol, 2.60 eq) synthesized in Example 8-2, tert-butyl N-(2-aminoethyl)carbamate (170.00 mg, 1.06 mmol, 1.00 eq), and n-BuOH (11 mL) were added, and then stirring and reflux were performed. After 48 hours, the mixture was concentrated in vacuo at 80°C and purified using a silica column with methylene chloride:methanol:ammonium hydroxide (15:1:0.1) to obtain 2-hexyloctyl 8-(3-((8-((2-hexyloctyl)oxy)-8-oxooctyl)amino)-3-oxopropyl)-2,2-dimethyl-4,11-dioxo-3-oxa-5,8,12-triazaicosan-20-oate with low purity. Subsequently, the synthesis of the compound of formula O was conducted without further purification process. In a 25 mL 1-neck RBF, 2-hexyloctyl 8-(3-((8-((2-hexyloctyl)oxy)-8-oxooctyl)amino)-3-oxopropyl)-2,2-dimethyl-4,11-dioxo-3-oxa-5 ,8,12-triazicosane-20-oate and methylene chloride (4.4 mL) were added, and trifluoroacetic acid (0.4 mL) was added dropwise. After stirring at room temperature for 24 hours, the mixture in the reactor was extracted with a saturated aqueous solution of sodium bicarbonate, and the organic layer was dried over sodium sulfate and filtered. The filtrate was concentrated in vacuo and purified using a silica column with methylene chloride:methanol:ammonium hydroxide (10:1:0.1) to obtain the compound of formula O (225.5 mg, two steps 24.2%).
¹H-NMR (400 MHz, CDCl₃) δ 6.81 (t, 2H), 3.96 (d, 4H), 3.15 (q, 4H), 3.03 (t, 2H), 2.63-2.66 (m, 6H), 2.35 (t, 4H), 2.29 (t, 4H), 1.59-1.62 (m, 6H), 1.46-1.50 (m, 4H), 1.27-1.39 (s, 55H), 0.87-0.92 (m, 12H)

### Example 16

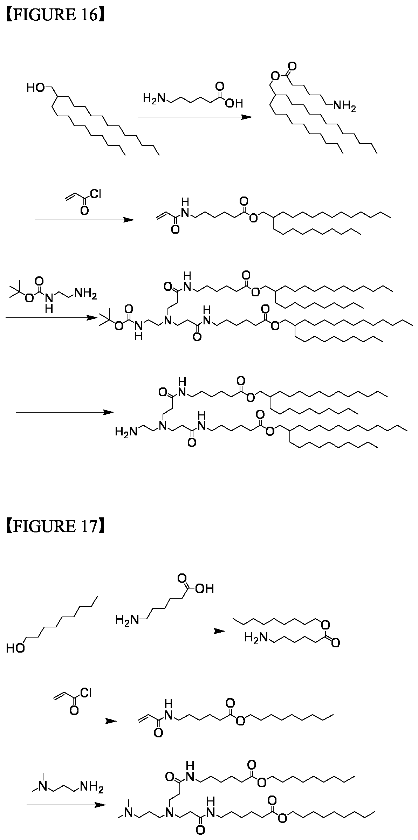

### 16-1. The compound of the following formula P was prepared according to the synthesis scheme shown in Figure 16.

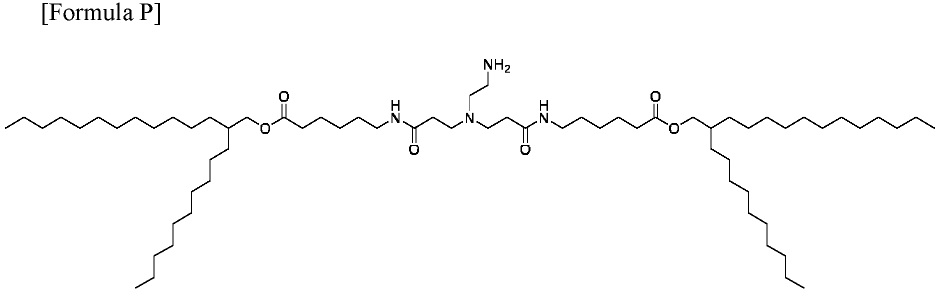

### 16-2. Synthesis of the compound of formula P

In a 100 mL 3-neck RBF, 2-decyltetradecyl 6-acrylamidohexanoate (891.15 mg, 1.71 mmol, 2.40 eq) synthesized in Example 9-2, tert-butyl N-(2-aminoethyl)carbamate (114.00 mg, 0.71 mmol, 1.00 eq), and n-BuOH (6.2 mL) were added, and then stirring and reflux were performed. After 48 hours, the mixture was concentrated in vacuo at 80°C and purified using a silica column with methylene chloride:methanol:ammonium hydroxide (20:1:0.1) to obtain 2-decyltetradecyl 8-(3-((6-((2-decyltetradecyl)oxy)-6-oxohexyl)amino)-3-oxopropyl)-2,2-dimethyl-4,11-dioxo-3-oxa-5,8,12-triazaoctadecan-18-oate with low purity. Subsequently, the synthesis of the compound of formula P was conducted without further purification process. In a 25 mL 1-neck RBF, 2-decyltetradecyl 8-(3-((6-((2-decyltetradecyl)oxy)-6-oxohexyl)amino)-3-oxopropyl)-2,2-dimethyl-4,11-dioxo-3-oxa-5,8,12-triazaoctadecan-18-oate and methylene chloride (6.0 mL) were added, and trifluoroacetic acid (0.6 mL) was added dropwise. After stirring at room temperature for 24 hours, the mixture in the reactor was extracted with a saturated aqueous solution of sodium bicarbonate, and the organic layer was dried over sodium sulfate and filtered. The filtrate was concentrated in vacuo and purified using a silica column with methylene chloride:methanol:ammonium hydroxide (15:1:0.1) to obtain the compound of chemical formula P (157.9 mg, two steps 20.1%).
¹H-NMR (400 MHz, CDCl₃) δ 6.98 (t, 2H), 3.96 (d, 4H), 3,16 (q, 4H), 3.01 (t, 2H), 2.65 (t, 6H), 2.36 (t, 4H), 2.30 (t, 4H), 1.60-1.66 (m, 7H), 1.48-1.54 (m, 5H), 1.20-1.40 (m, 83H), 0.90-0.95 (m, 12H)

### Example 17

### 17-1. The compound of the following formula Q was prepared according to the synthesis scheme shown in Figure 17.

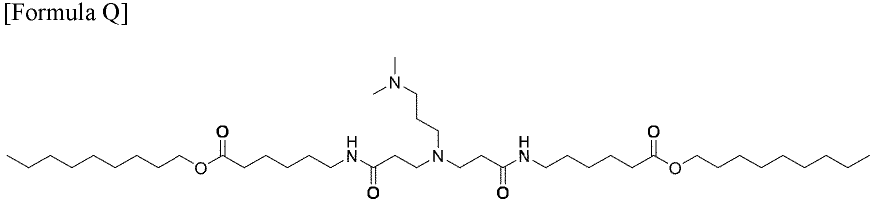

### 17-2. Synthesis of the compound of formula Q

In a 100 mL 3-neck RBF, nonyl 6-acrylamidohexanoate (3000.00 mg, 9.63 mmol, 2.60 eq) synthesized in Example 11-2, N,N-dimethylpropanediamine (378.53 mg, 1.00 mmol, 1.00 eq), and n-BuOH (18 mL) were added, and then stirring and reflux were performed. After 24 hours, the mixture was concentrated in vacuo at 80°C and purified using a silica column with methylene chloride:methanol:ammonium hydroxide (7:1:0.1) to obtain the compound of formula Q (1257.6 mg, yield: 47%).
¹H-NMR (400 MHz, CDCl₃) δ 4.05 (t, 4H), 3.22 (q, 4H), 2.73 (t, 4H), 2.50 (t, 2H), 2.25-2.47 (m, 10H), 2.23 (s, 6H), 1.60-1.67 (m, 10H), 1.48-1.58 (m, 4H), 1.27-1.38 (m, 28H), 0.88 (t, 6H)

### Example 18

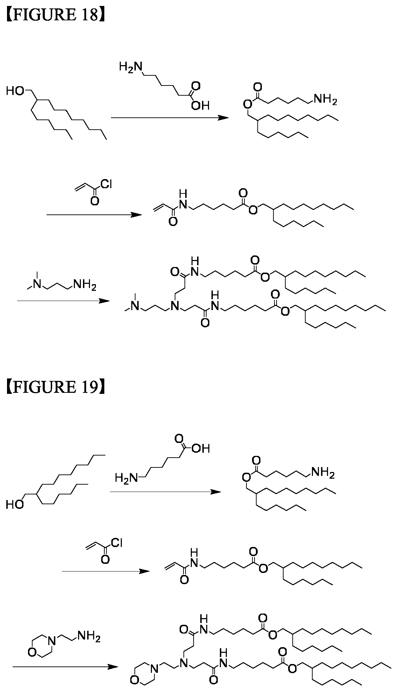

### 18-1. The compound of the following formula R was prepared according to the synthesis scheme shown in Figure 18.

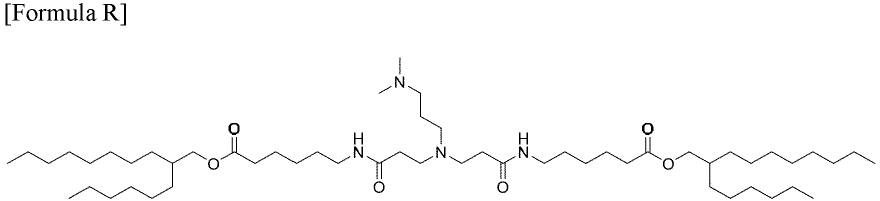

### 18-2. Synthesis of the compound of formula R

In a 100 mL 3-neck RBF, 2-hexyldecyl 6-acrylamidohexanoate (1500.00 mg, 3.67 mmol, 2.60 eq) synthesized in Example 2-2, N,N-dimethylpropanediamine (143.90 mg, 1.41 mmol, 1.00 eq), and n-BuOH (7 mL) were added, and then stirring and reflux were performed. After 24 hours, the mixture was concentrated in vacuo at 80°C and purified using a silica column with methylene chloride: methanol: ammonium hydroxide (10:1:0.1) to obtain the compound of formula R (476.10 mg, yield: 37%).
¹H-NMR (400 MHz, CDCl₃) δ 6.88 (t, 2H), 3.97 (d, 4H), 3.21 (q, 4H), 2.72 (t, 4H), 2.49 (t, 2H), 2.29-2.35 (m, 10H), 2.22 (s, 6H), 1.61-1.67 (m, 9H), 1.49-1.55 (m, 4H), 1.27-1.40 (m, 50H), 0.88 (t, 12H)

### Example 19

### 19-1. The compound of the following formula S was prepared according to the synthesis scheme shown in Figure 19.

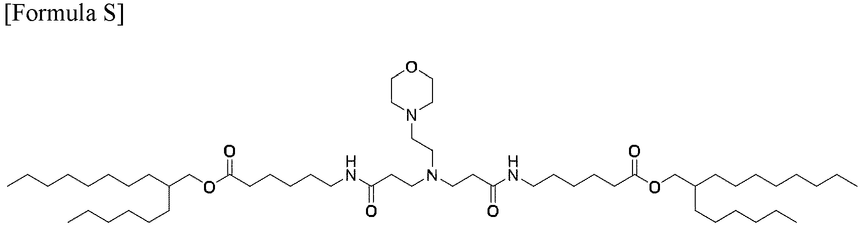

### 19-2. Synthesis of the compound of formula S

In a 100 mL 3-neck RBF, 2-hexyldecyl 6-acrylamidohexanoate (2000.00 mg, 4.88 mmol, 3.00 eq) synthesized in Example 2-2, 2-morpholinoethan-1-ol (210.00 mg, 1.63 mmol, 1.00 eq), and n-BuOH (20 mL) were added, and then stirring and reflux were performed. After 48 hours, the mixture was concentrated in vacuo at 80°C, and purified using a silica column with methylene chloride:methanol:ammonium hydroxide (15:1:0.1) to obtain the compound of chemical formula S (596.00 mg, yield: 39%).
¹H-NMR (400 MHz, CDCl₃) δ 6.82 (t, 2H), 3.96 (d, 4H), 3.69 (t, 4H), 3.22 (q, 4H), 2.76 (t, 3H), 2.58 (t, 2H), 2.43-2.49 (m, 6H), 2.29-2.34 (m, 7H), 1.61-1.67 (m, 6H), 1.38-1.54 (m, 4H), 1.27-1.38 (m, 52H), 0.88 (t, 12H)

### Example 20

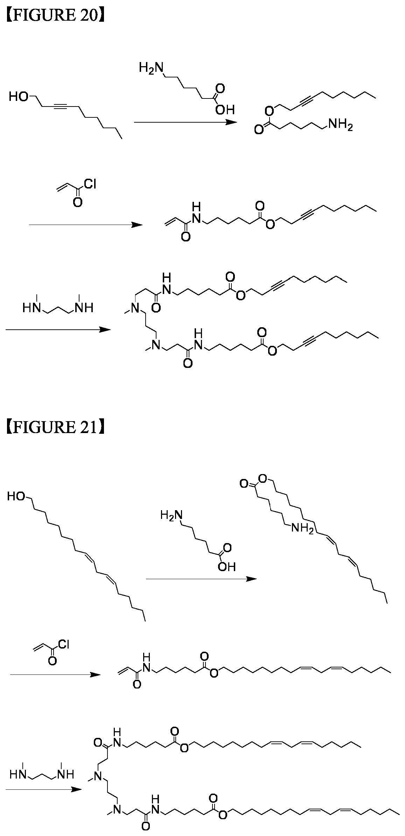

### 20-1. The compound of the following formula T was prepared according to the synthesis scheme shown in Figure 20.

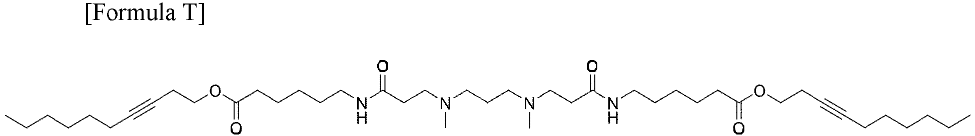

### 20-2. Synthesis of dec-3-yn-1-yl 6-acrylamidohexanoate

In a 500 mL 3-neck RBF, 6-aminohexanoic acid (3.06 g, 23.34 mmol, 1.20 eq), dec-3-yn-1-ol (3.00 g, 19.45 mmol, 1.00 eq), p-toluenesulfonic acid monohydrate (5.55 g, 29.17 mmol, 1.50 eq), and cyclohexane (130 mL) were added, and a Dean-Stark trap and a condenser were installed and then stirring and reflux were performed. After 24 hours, the mixture was cooled to room temperature, concentrated in vacuo, extracted with methylene chloride and NaOH aqueous solution, and the organic layer was dried over sodium sulfate and filtered. The residue was concentrated in vacuo to obtain dec-3-yn-1-yl 6-aminohexanoate with low purity. Without further purification, in a 500 mL 3-neck RBF, previously obtained dec-3-yn-1-yl 6-aminohexanoate, methylene chloride (130 mL), and triethylamine (4.33 g, 42.79 mmol, 2.20 eq) were added and cooled to 0 °C, and acryloyl chloride (1.94 g, 21.39 mmol, 1.10 eq) was added dropwise. The temperature of the reactor was raised to room temperature (20-25°C) and stirring was performed. After 18 hours, the mixture in the reactor was extracted with an HCl aqueous solution, and the organic layer was dried over sodium sulfate and filtered. The filtrate was concentrated in vacuo and purified using a silica column with EtOAc:hexane (1:2) to obtain dec-3-yn-1-yl 6-acrylamidohexanoate (765.60 mg, yield: 12%).
¹H-NMR (400 MHz, CDCl₃) δ 6.29 (d, 1H), 6.11 (m, 1H), 5.64 (d, 2H), 4.15 (t, 2H), 3.36 (t, 2H), 2.49 (m, 2H), 2.35 (t, 2H), 2.15 (t, 2H), 1.67 - 1.26 (m, 14H), 0.88 (t, 3H)

### 20-3. Synthesis of the compound of formula T

In a 100 mL 3-neck RBF, dec-3-yn-1-yl 6-acrylamidohexanoate (300.00 mg, 0.94 mmol, 2.20 eq), N,N'-dimethyl-1,3-propanediamine (43.80 mg, 0.43 mmol, 1.00 eq), and n-BuOH (5 mL) were added, and then stirring and reflux were performed. After 24 hours, the mixture was concentrated in vacuo at 80°C and purified using a silica column with methylene chloride: methanol: ammonium hydroxide (10:1:0.1) to obtain the compound of formula T (165.3 mg, yield: 52%).
¹H-NMR (400 MHz, CDCl₃) δ 7.90 (s, 2H), 4.13 (t, 4H), 3.22 (q, 4H), 2.62 (t, 4H), 2.48 (m, 4H), 2.42 (m, 4H), 2.36 (t, 4H), 2.32 (t, 4H), 2.25 (s, 6H), 2.15 (m, 4H), 1.63-1.79 (m, 7H), 1.40-1.60 (m, 9H), 1.25-1.38 (m, 8H), 0.90 (t, 6H)

### Example 21

### 21-1. The compound of the following formula U was prepared according to the synthesis scheme shown in Figure 21.

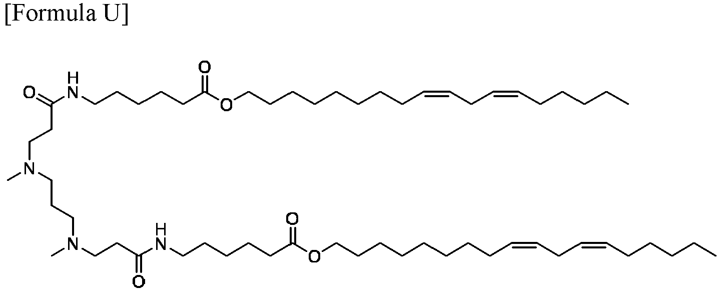

### 21-2. Synthesis of (9Z,12Z)-octadeca-9,12-dien-1-yl 6-acrylamidohexanoate

In a 100 mL 3-neck RBF, 6-aminohexanoic acid (295.37 mg, 2.25 mmol, 1.20 eq), (9Z,12Z)-octadeca-9,12-dien-1-ol (500.00 mg, 1.88 mmol, 1.00 eq), p-toluenesulfonic acid monohydrate (642.47 mg, 3.38 mmol, 1.80 eq), and cyclohexane (20 mL) were added, and a Dean-Stark trap and a condenser were installed and then stirring and reflux were performed. After 24 hours, the mixture was cooled to room temperature, concentrated in vacuo, extracted with methylene chloride and NaOH aqueous solution, and the organic layer was dried over sodium sulfate and filtered. The filtrate was concentrated in vacuo to obtain (9Z,12Z)-octadeca-9,12-dien-1-yl 6-aminohexanoate with low purity. Without further purification, previously obtained (9Z,12Z)-octadeca-9,12-dien-1-yl 6-aminohexanoate, methylene chloride (20 mL), and triethylamine (417.72 mg, 4.13 mmol, 2.20 eq) were added to a 100 mL 3-neck RBF and cooled to 0°C, and acryloyl chloride (186.82 mg, 2.06 mmol, 1.10 eq) was added dropwise. The temperature of the reactor was raised to room temperature (20-25°C) and stirring was performed. After 18 hours, the mixture in the reactor was extracted with an aqueous HCl solution, and the organic layer was dried over sodium sulfate and filtered. The filtrate was concentrated in vacuo and purified using a silica column with EtOAc:hexane (1:2) to obtain (9Z,12Z)-octadeca-9,12-dien-1-yl 6-acrylamidohexanoate (579.9 g, yield: 71%).
¹H NMR (400 MHz, CDCl₃) δ 6.27 (dd, 1H), 6.05-6.11 (m, 1H), 5.62-5.64 (m, 2H), 5.34-5.38 (m, 4H), 4.05 (t, 2H), 3.33 (q, 2H), 2.76 (t, 2H), 2.29 (t, 2H), 2.05 (q, 4H), 1.56-1.67 (m, 6H), 1.28-1.39 (m, 18H), 0.89 (t, 3H)

### 21-3. Synthesis of the compound of formula U

In a 100 mL 3-neck RBF, (9Z,12Z)-octadeca-9,12-dien-1-yl 6-acrylamidohexanoate (305.258 mg, 0.70 mmol, 2.40 eq), N,N'-dimethyl-1,3-propanediamine (30.00 mg, 0.29 mmol, 1.00 eq), and n-BuOH (3 mL) were added, and then stirring and reflux were performed. After 24 hours, the mixture was concentrated in vacuo at 80°C and purified using a silica column with methylene chloride: methanol: ammonium hydroxide (10:1:0.1) to obtain the compound of formula U (185.10 mg, yield: 65%).
¹H NMR (400 MHz, CDCl₃) δ 7.85 (s, 2H), 5.34-5.38 (m, 8H), 4.05 (s, 4H), 3.22 (q, 4H), 2.80 (t, 4H), 2.60 (t, 4H), 2.42 (t, 4H), 2.36 (t, 4H), 2.28 (t, 4H), 2.03 (s, 6H), 2.05 (q, 8H), 1.60-1.68 (m, 10H), 1.49-1.60 (m, 4H), 1.27-1.37 (m, 36H), 0.89 (t, 3H)

### Example 22

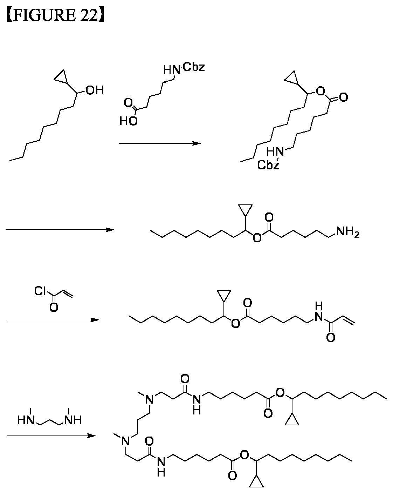

### 22-1. The compound of the following formula V was prepared according to the synthesis scheme shown in Figure 22.

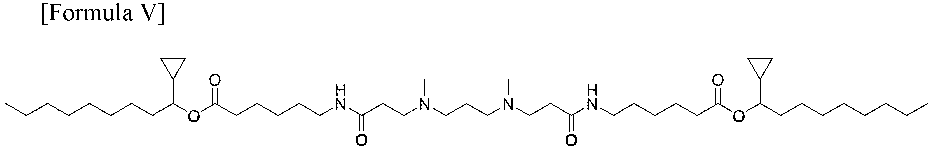

### 22-2. Synthesis of 1-cyclopropylnonyl 6-(((benzoyloxy)carbonyl)amino)hexanoate

In a 500 mL 3-neck RBF, 1-cyclopropylnonan-1-ol (8.68 g, 47.1 mmol, 1.00 eq) and methylene chloride (170 mL) were added at 25°C. 1-Ethyl-3-(3-dimethylaminopropyl)carbodiimide (EDCI) (11.7 g, 61.3 mmol, 1.30 eq) and triethylamine (Et3N) (9.54 g, 94.2 mmol, 2.00 eq) were added to the mixture. 6-(((benzyloxy)carbonyl)amino)hexanoic acid (15.0 g, 56.5 mmol, 1.20 eq) and 4-dimethylaminopyridine (DMAP) (1.15 g, 9.42 mmol, 0.20 eq) were added. The mixture was purged with nitrogen (N₂) three times. The mixture was stirred at 25°C for 16 hours. The reaction mixture was diluted with water (200 mL) and extracted with 600 mL of methylene chloride (200 mL x 3). The methylene chloride layer was collected, dried over sodium sulfate, filtered, and the filtrate was concentrated under reduced pressure to obtain the residue. The residue was purified by column chromatography (SiO₂, petroleum ether (PE)/ethyl acetate (EtOAc) = 10/1 to 1/100). 1-Cyclopropylnonyl 6-(((benzoyloxy)carbonyl)amino)hexanoate (9.00 g, 20.9 mmol, 44.3% yield) was obtained as a colorless oil.
¹H NMR (400 MHz, CDCl₃) δ 4.27 (td, 1H), 3.41 (t, 2H), 2.31 (t, 2H), 1.89 - 1.74 (m, 2H), 1.68 - 1.60 (m, 4H), 1.43 (s, 2H), 1.38 - 1.25 (m, 16H), 0.99 - 0.92 (m, 1H), 0.89 (t, 3H), 0.59 - 0.51 (m, 1H), 0.49 - 0.42 (m, 1H), 0.38 (qd, 1H), 0.31 - 0.21 (m, 1H)

### 22-3. Synthesis of 1-cyclopropylnonyl 6-aminohexanoate

Under argon (Ar) atmosphere, palladium catalyst (Pd/C) (2.22 g, 2.09 mmol, 10% purity, 0.10 eq) was added to a 35 mL portion of a cylindrical flask. Tetrahydrofuran (THF) (90 mL) was added to the top of the cylindrical flask. To the mixture, 1-cyclopropylnonyl 6-(((benzoyloxy)carbonyl)amino)hexanoate (9.00 g, 20.9 mmol, 1.00 eq) was added. Hydrogen (H₂) was charged to 50 psi. The mixture was stirred at 50°C for 16 hours. The Pd/C filter cake was safely filtered and collected. The filtrate was concentrated under pressure to obtain the residue. The residue was purified by column chromatography (SiO₂, methylene chloride/methanol = 10/1 to 1/100) to obtain 1-cyclopropylnonyl 6-aminohexanoate (6.00 g, 20.2 mmol, 96.7% yield) as a yellow oil.
¹H NMR (400 MHz, CDCl₃) δ 4.27 (td, 1H), 2.71 (t, 2H), 2.32 (t, 2H), 1.71 - 1.60 (m, 5H), 1.53 - 1.43 (m, 2H), 1.42 - 1.18 (m, 15H), 1.00 - 0.92 (m, 1H), 0.89 (t, 3H), 0.59 - 0.50 (m, 1H), 0.49 - 0.42 (m, 1H), 0.38 (qd, 1H), 0.33 - 0.18 (m, 1H)

### 22-4. Synthesis of 1-cyclopropylnonyl 6-acrylamidohexanoate

In a 100 mL 3-neck RBF, methylene chloride (30 mL) and 1-cyclopropylnonyl 6-aminohexanoate (3.00 g, 10.1 mmol, 1.00 eq) were added. To the mixture, triethylamine (TEA) (4.59 g, 45.4 mmol, 4.50 eq) was added at 0°C. To the mixture, acryloyl chloride (1.37 g, 15.1 mmol, 1.50 eq) was added dropwise at 0°C. The mixture was stirred at 0°C for 2 hours and then slowly returned to room temperature. The reaction mixture was neutralized by the addition of water (40 mL) at 20°C and extracted with methylene chloride (50 mL x 3). The combined organic layers were dried over sodium sulfate, filtered, and the filtrate was concentrated under reduced pressure to obtain the residue. The residue was purified by column chromatography (SiO₂, PE/EtOAc=10/1 to 1/100). 1-Cyclopropylnonyl 6-acrylamidohexanoate (2.60 g, 7.40 mmol, 73.3% yield) was obtained as a yellow oil.
¹H NMR (400 MHz, CDCl₃) *δ* 6.34 - 6.21 (m, 1H), 6.15 - 5.98 (m, 1H), 5.64 (br dd, 1H), 4.27 (td, 1H), 3.41 - 3.08 (m, 2H), 2.40 - 2.22 (m, 2H), 1.72 - 1.61 (m, 4H), 1.58 (s, 6H), 1.45 - 1.35 (m, 2H), 1.27 (br s, 9H), 1.00 - 0.91 (m, 1H), 0.89 (t, 3H), 0.61 - 0.51 (m, 1H), 0.50 - 0.41 (m, 1H), 0.37 (qd, 1H), 0.30 - 0.20 (m, 1H)

### 22-5. Synthesis of the compound of formula V

In a 50 mL 3-neck RBF, a mixture of dimethyl sulfoxide (DMSO) (3.5 mL) and water (3.5 mL), and 1-cyclopropylnonyl 6-acrylamidohexanoate (0.70 g, 1.99 mmol, 1.00 eq) were added. To the mixture, TEA (134 mg, 1.33 mmol, 0.67 eq) was added. To the mixture, N¹,N³-dimethylpropane-1,3-diamine (67.8 mg, 663 µmol, 0.33 eq) was added. The mixture was purged three times with N₂. The mixture was stirred at 100°C for 48 hours. The reaction mixture was concentrated under reduced pressure to obtain a residue. The residue was purified by column chromatography (SiO₂, methylene chloride/methanol=10/1) to obtain 0.3 g of a compound of formula V with relatively low purity. It was purified by reverse-phase HPLC (column: Phenomenex luna C18 15025 mm 10 µm; mobile phase: [water (FA)-ACN]; slope: 40%-70% B for 10 min) to obtain the compound of formula V (0.15 g, 186 µmol, 28.1% yield, 96.1% purity) as a yellow oil.
¹H NMR (400 MHz, CDCl₃) *δ* 7.91 (br s, 2H), 4.26 (td, 2H), 3.31 - 3.17 (m, 4H), 2.61 (t, 4H), 2.41 (t, 4H), 2.38 - 2.33 (m, 4H), 2.33 - 2.27 (m, 4H), 2.25 (s, 6H), 1.72 - 1.62 (m, 10H), 1.56 - 1.48 (m, 4H), 1.41 - 1.25 (m, 28H), 1.01 - 0.92 (m, 2H), 0.89 (t, 6H), 0.59 - 0.50 (m, 2H), 0.49 - 0.41 (m, 2H), 0.36 (qd, 2H), 0.31 - 0.21 (m, 2H)

### [Preparation Example of a composition for drug delivery]

### 1. Raw material preparation

According to the following table, the materials required for preparing the formulation were dissolved in each dilution solvent and prepared at the required concentration. When dissolving, the materials were kept at room temperature and then the solvent was added to dissolve them.

| | **Materials** | **Concentration for use** |
|---|---|---|
| 1 | mRNA | 1 mg/mL |
| 2 | Each compound of formulas A to V | 10 - 20 mg/mL |
| 3 | DMG-PEG (1,2-dimyristoyl-rac-glycero-3-methoxypolyethylene glycol-2000) | 5 - 10 mg/mL |
| 4 | Distearoyl phosphatidylcholine (DSPC) | 10 - 20 mg/mL |
| 5 | Cholesterol | 10 - 20 mg/mL |

### 2. Raw material mixing

The required amounts of the raw materials were mixed in order to meet the N/P ratio (amine group of lipid/phosphate group of mRNA) of 10 and each compound of formulas A to V:DMG-PEG:DSPC:Cholesterol=50:1.5:10:38.5. Ethanol was added to the ethanol layer so that the molecular total of all raw materials was within 12.5 mM, and the aqueous phase and ethanol phase were mixed maintaining a volume ratio of 3:1. After the mixing, to reduce the total ethanol content, buffer exchange was performed as follows: The mixture solution was concentrated by centrifugation at 4,000 rpm to 1/3 using an Amicon-Ultra tube filter (Merk Millipore, UFC505096 or UFC805024, pore size: 50K, volume: 0.5 mL or 4 mL), then diluted with three times the volume of PBS and centrifuged to concentrate. This process was repeated six times to exchange the buffer.

The more concrete procedure is as follows:
1) Two autoclaved tubes were prepared (tubes (A) and (B)).
2) In tube (A), each compound of formulas A to V, DSPC, cholesterol and DMG-PEG in molar quantities calculated according to the experimental conditions were sequentially added and mixed by vortexing.
3) In the ethanol phase, when necessary, ethanol was added so that the molecular total of all raw materials was within 12.5 mM.
4) In tube (B), mRNA and 20 mM sodium acetate buffer (pH 4.6) (=prepared by diluting with 3M sodium acetate buffer to 20 mM and titrating to pH 4.6 using 1M HCl) were mixed. At that time, the ratio was calculated and added so that the aqueous phase was total three times the amount of the ethanol phase.
5) Mixing of Tube (A) and Tube (B) was performed using a Microfluidics (Ignite, Precision Nanosystem) device. Microfluidics operating conditions were FRR (Flow Rate Ratio) of C:R=3:1 and TRR (Total Flow Rate) of 12 mL/min.
6) The resulting mixture from step 5) was concentrated by centrifugation at 4,000 rpm to 1/3 using an Amicon-Ultra tube filter (50K), then diluted 3-fold with PBS and concentrated by centrifugation. This process was repeated 6 times for concentration to the desired volume.

### 3. Evaluation of the properties of the formulation

1) For the prepared formulation, particle characteristics (i.e., Zeta-average particle size (Z-average), polydispersity index (PDI), and zeta-potential) were confirmed using a particle size analyzer (Dynamic Light Scattering, DLS), and the results are shown in Table 1 below.
2) For the prepared formulation, mRNA encapsulation efficiency was confirmed through Ribo-green assay, and the results are shown in Table 1 below.

**[Table 1]**

| **Lipid compound** | **Z-average (nm)** | **PDI (PI)** | **Zeta-potential (mV)** | **Encapsulation efficiency (%)** |
|---|---|---|---|---|
| Example 1 | 123.7 ± 1.41 | 0.158 ± 0.023 | 17.47 ± 0.987 | > 95.1 |
| Example 2 | 126.8 ± 2.69 | 0.196 ± 0.009 | 10.80 ± 1.192 | > 96.0 |
| Example 3 | 128.2 ± 5.293 | 0.441 ± 0.045 | 12.91 ± 1.813 | > 93.75 |
| Example 4 | 147.8 ± 0.979 | 0.231 ± 0.012 | -7.87 ± 0.754 | > 93.75 |
| Example 5 | 137.5 ± 1.364 | 0.156 ± 0.009 | -6.66 ± 0.365 | > 96.9 |
| Example 6 | 125.9 ± 1.325 | 0.190 ± 0.011 | -6.02 ± 2.949 | > 87.5 |
| Example 7 | 118.6 ± 2.099 | 0.215 ± 0.018 | 2.04 ± 0.365 | > 93.75 |
| Example 8 | 110.3 ± 1.700 | 0.188 ± 0.007 | 4.83 ± 0.301 | > 99.2 |
| Example 9 | 129.3 ± 0.595 | 0.180 ± 0.007 | -0.55 ± 1.201 | > 93.75 |
| Example 10 | 118.2 ± 1.473 | 0.171 ± 0.006 | 6.72 ± 0.073 | > 94.1 |
| Example 11 | 180.3 ± 0.919 | 0.160 ± 0.005 | 11.26 ± 1.701 | > 93.75 |
| Example 12 | 120.7 ± 1.177 | 0.180 ± 0.028 | -18.47 ± 1.042 | > 93.75 |
| Example 13 | 117.8 ± 1.659 | 0.192 ± 0.016 | -16.60 ± 1.990 | > 93.75 |
| Example 14 | 113.8 ± 0.502 | 0.166 ± 0.027 | -10.39 ± 1.994 | > 93.75 |
| Example 15 | 127.0 ± 0.325 | 0.186 ± 0.015 | -0.40 ± 0.239 | > 93.75 |
| Example 16 | 128.9 ± 4.714 | 0.128 ± 0.015 | 0.50 ± 3.461 | > 93.75 |
| Example 17 | 127.3 ± 0.955 | 0.167 ± 0.009 | 17.22 ± 3.314 | > 93.75 |
| Example 18 | 118.6 ± 0.565 | 0.199 ± 0.007 | 10.96 ± 1.293 | > 93.75 |
| Example 19 | 146.7 ± 1.226 | 0.150 ± 0.022 | -9.39 ± 0.137 | > 93.75 |
| Example 20 | 145.0 ± 1.225 | 0.080 ± 0.004 | -22.2 ± 0.634 | > 93.75 |
| Example 21 | 120.0 ± 4.251 | 0.350 ± 0.009 | 4.43 ± 0.746 | > 93.50 |
| Example 22 | 121.0 ± 4.06 | 0.3042 ± 0.026 | 3.56 ± 3.67 | > 93.20 |

## Claims

1. A lipid having a structure selected from the following, or an ionized form thereof: 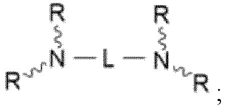 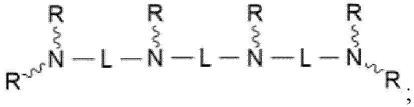 and 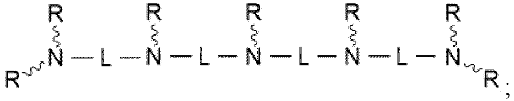 wherein, in each of the above structures,
at least two of R groups are Rx, and other R groups are Ry, wherein
each Rx is independently selected from 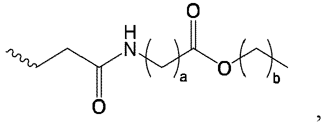 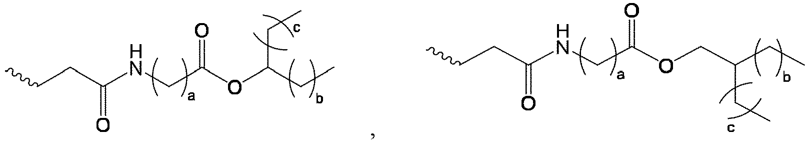 and 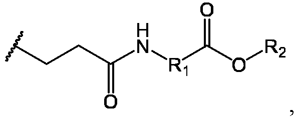 where each of a, b and c is independently an integer of from 2 to 20, R₁ is substituted or unsubstituted, saturated or unsaturated divalent hydrocarbon group, R₂ is substituted or unsubstituted, unsaturated monovalent hydrocarbon group, and 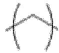 represents substituted or unsubstituted methylene group, and
each Ry is independently H, or substituted or unsubstituted alkyl group, where two Ry groups that are not H may be connected together with nitrogen atom to which they are attached, to form a ring structure; and
each L is independently substituted or unsubstituted alkylene group, and may have in its structure optionally ether bond (-O-), thioether bond (-S-) or disulfide bond (-S-S-).

2. The lipid according to Claim 1, wherein
each Rx is independently selected from 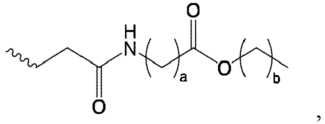 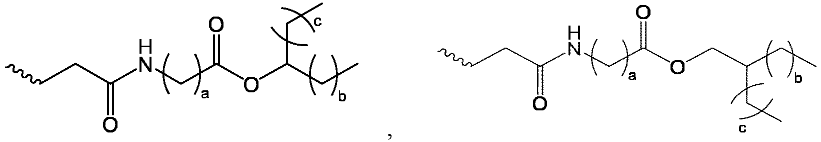 and 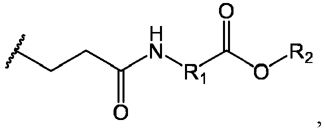 where each of a, b and c is independently an integer of from 2 to 20, R₁ is substituted or unsubstituted, saturated or unsaturated divalent C₁₋₁₂ hydrocarbon group, R₂ is substituted or unsubstituted, unsaturated monovalent C₂₋₂₄ hydrocarbon group, and 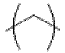 represents substituted or unsubstituted methylene group;
each Ry is independently H or C₁₋₂₀ alkyl group, where the alkyl group is independently unsubstituted, or substituted with one or more selected from -OH, C₁₋₂₀ alkyl, C₁₋₂₀ alkoxy, -NH₂, -NH(C₁₋₂₀ alkyl), -N(C₁₋₂₀ alkyl)₂, optionally substituted C₃₋₂₀ carbocyclic group and optionally substituted C₃₋₂₀ heterocyclic group, where two Ry groups that are not H may be connected together with nitrogen atom to which they are attached, to form a ring structure; and
each L is independently C₁₋₂₀ alkylene group, each of which is independently unsubstituted, or substituted with one or more selected from -OH, C₁₋₂₀ alkyl, C₁₋₂₀ alkoxy, -NH₂, - NH(C₁₋₂₀ alkyl), -N(C₁₋₂₀ alkyl)₂, optionally substituted C₃₋₂₀ carbocyclic group and optionally substituted C₃₋₂₀ heterocyclic group.

3. The lipid according to Claim 2, wherein
each Rx is independently selected from 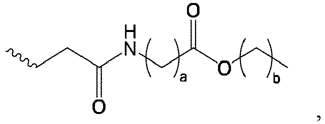 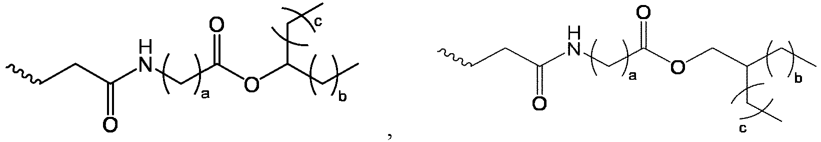 and 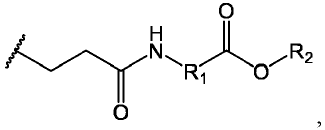 where each of a, b and c is independently an integer of from 2 to 15, R₁ is substituted or unsubstituted, saturated or unsaturated divalent C₁₋₁₂ hydrocarbon group, R₂ is substituted or unsubstituted, unsaturated monovalent C₂₋₂₄ hydrocarbon group, and 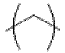 represents substituted or unsubstituted methylene group;
each Ry is independently H or C₁₋₁₀ alkyl group, where the alkyl group is independently unsubstituted, or substituted with one or more selected from -OH, C₁₋₁₀ alkyl, C₁₋₁₀ alkoxy, -NH₂, -NH(C₁₋₁₀ alkyl), -N(C₁₋₁₀ alkyl)₂, optionally substituted C₃₋₁₀ carbocyclic group and optionally substituted C₃₋₁₀ heterocyclic group, where two Ry groups that are not H may be connected together with nitrogen atom to which they are attached, to form a ring structure; and
each L is independently C₁₋₁₀ alkylene group, each of which is independently unsubstituted, or substituted with one or more selected from -OH, C₁₋₁₀ alkyl, C₁₋₁₀ alkoxy, -NH₂, -NH(C₁₋₁₀ alkyl), -N(C₁₋₁₀ alkyl)₂, optionally substituted C₃₋₁₀ carbocyclic group and optionally substituted C₃₋₁₀ heterocyclic group.

4. The lipid according to Claim 3, wherein
each Rx is independently selected from 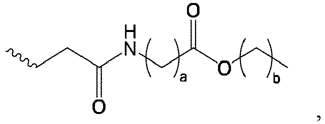 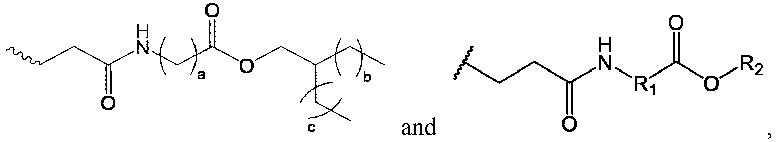 where each of a, b and c is independently an integer of from 3 to 12, R₁ is substituted or unsubstituted C₁₋₁₂ alkylene group, substituted or unsubstituted C₂₋₁₂ alkenylene group or substituted or unsubstituted C₂₋₁₂ alkynylene group, R₂ is substituted or unsubstituted C₂₋₂₄ alkenyl group or substituted or unsubstituted C₂₋₂₄ alkynyl group, and 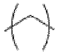 represents substituted or unsubstituted methylene group;
each Ry is independently H or C₁₋₆ alkyl group, where the alkyl group is independently unsubstituted, or substituted with one or more selected from -OH and -NH₂; and two Ry groups that are not H may be connected together with nitrogen atom to which they are attached, to form a ring structure; and
each L is independently unsubstituted C₁₋₆ alkylene group.

5. The lipid according to Claim 4, wherein the lipid is one having a structure selected from the following formulas A to V:
| Formula | Structure |
|---|---|
| A | 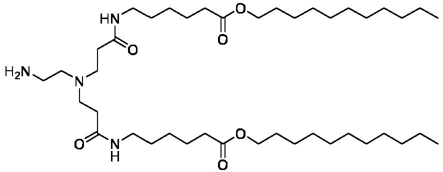 |
| B | 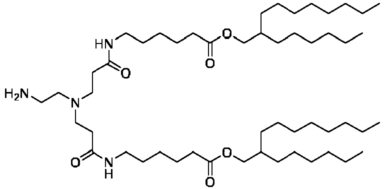 |
| C | 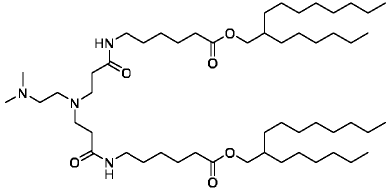 |
| D | 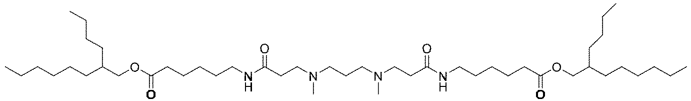 |
| E | 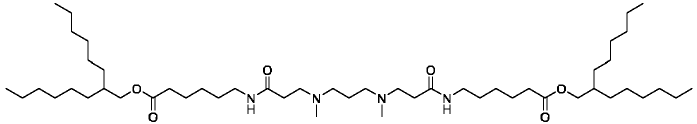 |
| F | 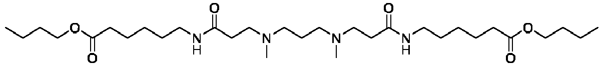 |
| G | 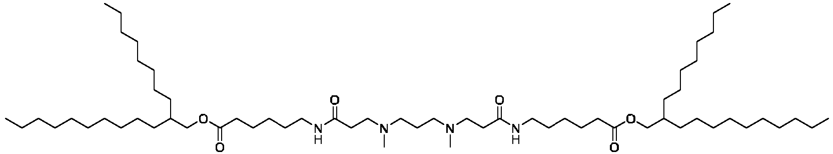 |
| H | 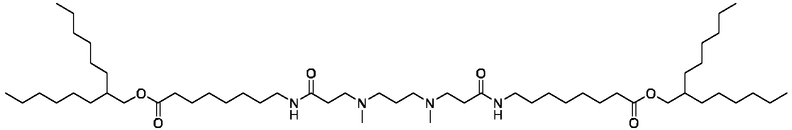 |
| I | 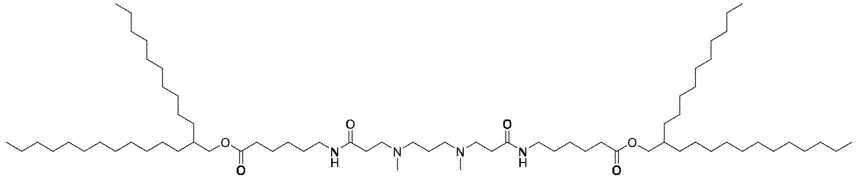 |
| J | 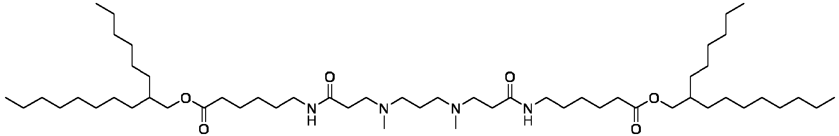 |
| K | 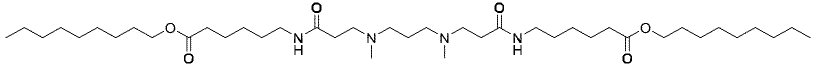 |
| L | 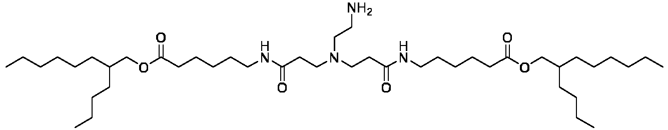 |
| M | 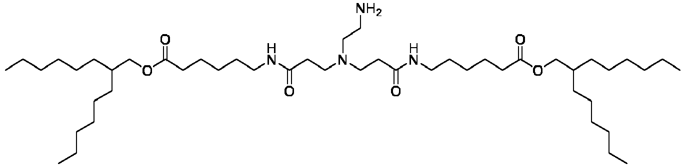 |
| N | 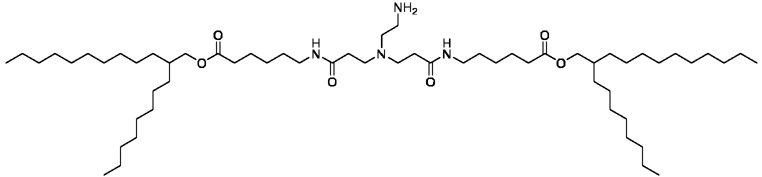 |
| O | 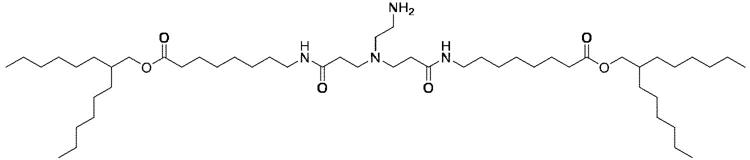 |
| P | 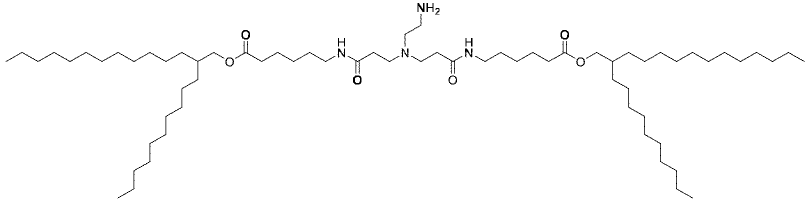 |
| Q | 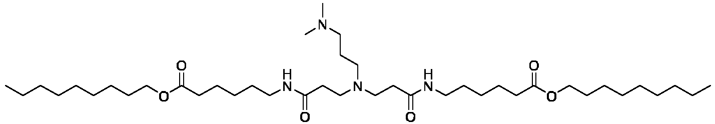 |
| R | 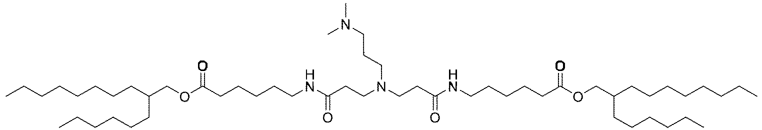 |
| S | 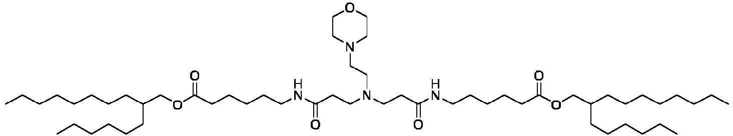 |
| T | 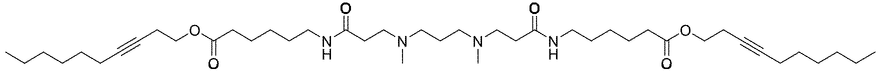 |
| U | 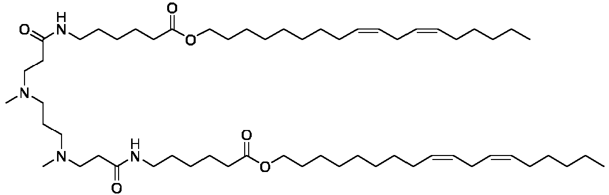 |
| V | 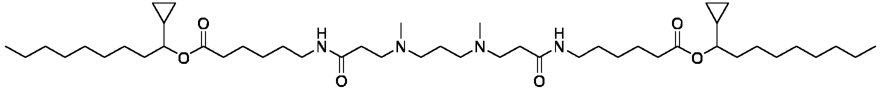 |

6. A method for preparing a lipid having a structure represented by formula (1-1), comprising steps of:
(1) reacting a compound of formula (a) with a compound of formula (b) to obtain a compound of formula (c);
(2) reacting a compound of formula (c) with a compound of formula (d) to obtain a compound of formula (e); and
(3) reacting a compound of formula (e) with a compound of formula (f) and deprotecting the reaction product:
[Formula a] H₂N-(CH₂)ₐ-C(=O)OH
[Formula b] OH-R'
[Formula c] H₂N-(CH₂)ₐ-C(=O)O-R' 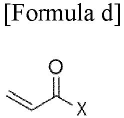
[Formula e] H₂C=CH-C(=O)-HN-(CH₂)ₐ-C(=O)O-R'
[Formula f] H₂N-(CH₂)₁₋₂₀-NH-C(=O)O-C(CH₃)₃
[Formula 1-1] H₂N-(CH₂)₁₋₂₀-N[-CH₂-CH₂-C(=O)-HN-(CH₂)ₐ-C(=O)O-R']₂
wherein,
each R' is independently 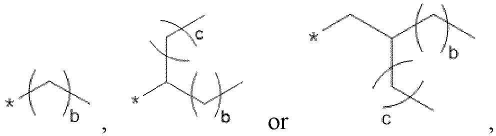 where * indicates the point of attachment to the adjacent oxygen atom, and 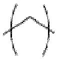 represents substituted or unsubstituted methylene group,
each of a, b and c is independently an integer of from 2 to 20, and
X is selected from the group consisting of F, CI, Br and I.

7. A method for preparing a lipid having a structure represented by formula (1-2), comprising a step of reacting a compound of formula (e) obtained in Claim 6 with a compound of formula (g):
[Formula e] H₂C=CH-C(=O)-HN-(CH₂)ₐ-C(=O)O-R'
[Formula g] H₂N-(CH₂)₁₋₂₀-N(C₁₋₂₀ alkyl)₂
[Formula 1-2] (C₁₋₂₀ alkyl)₂N-(CH₂)₁₋₂₀-N[-CH₂-CH₂-C(=O)-HN-(CH₂)ₐ-C(=O)O-R']₂
wherein,
each R' is independently 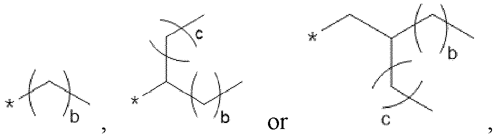 where * indicates the point of attachment to the adjacent oxygen atom, and 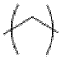 represents substituted or unsubstituted methylene group, and
each of a, b and c is independently an integer of from 2 to 20.

8. A method for preparing a lipid having a structure represented by formula (1-3), comprising a step of reacting a compound of formula (e) obtained in Claim 6 with a compound of formula (h):
[Formula e] H₂C=CH-C(=O)-HN-(CH₂)ₐ-C(=O)O-R'
[Formula h] (C₁₋₁₀ alkyl)-NH-(CH₂)₁₋₂₀-NH-(C₁₋₁₀ alkyl)
[Formula 1-3] A-N(C₁₋₁₀ alkyl)-(CH₂)₁₋₂₀-N(C₁₋₁₀ alkyl)-A
wherein,
each R' is independently 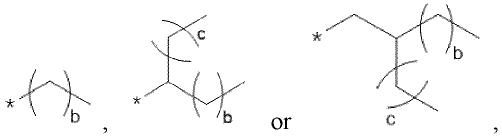 where * indicates the point of attachment to the adjacent oxygen atom, and 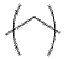 represents substituted or unsubstituted methylene group,
A is -CH₂-CH₂-C(=O)-HN-(CH₂)ₐ-C(=O)O-R',
each of a, b and c is independently an integer of from 2 to 20, and
X is selected from the group consisting of F, CI, Br and I.

9. A method for preparing a lipid having a structure represented by formula (1-4), comprising steps of:
(1) reacting a compound of formula (a) with a compound of formula (b') to obtain a compound of formula (c');
(2) reacting a compound of formula (c') with a compound of formula (d) to obtain a compound of formula (e'); and
(3) reacting a compound of formula (e') with a compound of formula (h):
[Formula a] H₂N-(CH₂)ₐ-C(=O)OH
[Formula b'] OH-R₂
[Formula c'] H₂N-R₁-C(=O)O-R₂ 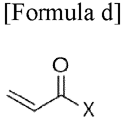
[Formula e'] H₂C=CH-C(=O)-HN-R₁-C(=O)O-R₂
[Formula h] (C₁₋₁₀ alkyl)-NH-(CH₂)₁₋₂₀-NH-(C₁₋₁₀ alkyl)
[Formula 1-4] A'-N(C₁₋₁₀ alkyl)-(CH₂)₁₋₂₀-N(C₁₋₁₀ alkyl)-A'
wherein,
A' is -CH₂-CH₂-C(=O)-HN-R₁-C(=O)O-R₂,
R₁ is independently substituted or unsubstituted, saturated or unsaturated divalent hydrocarbon group,
R₂ is independently substituted or unsubstituted, unsaturated monovalent hydrocarbon group,
a is an integer of from 2 to 20, and
X is selected from the group consisting of F, CI, Br and I.

10. A composition for drug delivery comprising the lipid of any one of claims 1 to 5.
